(19)

**Europäisches Patentamt**
**European Patent Office**
**Office européen des brevets**

(11)  **EP 4 670 732 A1**

(12)  **EUROPEAN PATENT APPLICATION**
published in accordance with Art. 153(4) EPC

(43) Date of publication:
**31.12.2025   Bulletin 2026/01**

(21) Application number: **24777774.1**

(22) Date of filing: **18.03.2024**

(51) International Patent Classification (IPC):
*A61K 39/00* (2006.01)   *A61K 35/766* (2015.01)
*A61K 38/19* (2006.01)   *A61K 38/20* (2006.01)
*A61P 35/00* (2006.01)   *A61P 35/02* (2006.01)

(52) Cooperative Patent Classification (CPC):
Y02A 50/30

(86) International application number:
**PCT/CN2024/082160**

(87) International publication number:
**WO 2024/198985 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **30.03.2023   CN 202310328769**

(71) Applicant: **Joint Biosciences (SH) Ltd.
Pudong New Area Shanghai 201318 (CN)**

(72) Inventors:
• **ZHOU, Guoqing
Shanghai 201318 (CN)**

• **MA, Liang
Shanghai 201318 (CN)**
• **TIAN, Ting
Shanghai 201318 (CN)**
• **CHENG, Longxin
Shanghai 201318 (CN)**
• **MA, Qibin
Shanghai 201318 (CN)**
• **ZHANG, Fan
Shanghai 201318 (CN)**

(74) Representative: **Dr. Gassner & Partner mbB
Wetterkreuz 3
91058 Erlangen (DE)**

(54)  **METHOD FOR TREATING TUMORS USING COMBINATION OF ONCOLYTIC VIRUS VACCINE AND IMMUNE CELLS**

(57)    The invention relates to the technical field of biomedicine, and specifically to a method for treating tumors using a combination of an oncolytic virus vaccine and immune cells. The method specifically comprises the following steps: treating a tumor by using a combination of immune cells and an oncolytic virus vaccine; the oncolytic virus vaccine comprising a recombinant oncolytic virus expressing a tumor antigen, and used for targeting tumor cells; the immune cells are embedded in an antigen receptor paired with the tumor antigen, and are used for killing or destroying tumor cells of a target; and the recombinant oncolytic virus comprises an M protein, G protein, N protein, P protein, and L protein, following site-directed mutagenesis. The combination of the oncolytic virus vaccine and the immune cells for killing or destroying the tumor antigen is used for attacking and killing tumor cells, and the tumor antigen expressed by the oncolytic virus vaccine can not only guide the immune cells to reach the center of a target tumor tissue, but the combination of the oncolytic virus and immune cells to kill tumor cells achieves a curative effect where 1+1 is greater than 2, with a maximum tumor cell killing rate being able to reach 100%.

FIG. 1

## Description

## TECHNICAL FIELD

The present application relates to the technical field of biomedicine and, in particular, to a method for treating tumors using a combination of an oncolytic virus vaccine and immune cells.

## BACKGROUND

Immune cell therapy is expected to be a breakthrough strategy to "cure" cancer. CAR-T (Chimeric Antigen Receptor T)-Cell Immunotherapy has achieved remarkable results in the treatment of various hematological tumors. So far, there are 7 CAR-T cell therapy products on the market around the world, including 3 products marketed in China.

Although CAR-T cell immunotherapy has been successful in treating hematological tumors, it still has many limitations in treating solid tumors. The following key factors lead to these limitations:

(1) Due to the heterogeneity of tumor-associated antigens of solid tumor cells, there are no antigen targets attacked by CAR-T cells; and
(2) CAR-T cells are not able to penetrate through solid tumor tissue microenvironment systems and ultimately reach the target tumor tissue.

Similarly, other immune cell therapies, including but not limited to TCR-T cells, $\gamma\delta$-T cells, Crispered-T cells, STAR-T cells, CAR-NK cells, CAR-M cells, including but not limited to immune cells derived from autologous cells, allogeneic cells or IPSC, also have these two major limitations.

In addition, even among patients whose treatment for hematological tumors is effective, a considerable proportion of patients will still relapse after treatment. After recurrence, patients will lose the previously treated targets in theCAR-T cell immunotherapy, so they can no longer be treated with the same target.

## SUMMARY

In order to further improve the curative effect of tumor cells, the present application provides a method for treating tumors using a combination of an oncolytic virus vaccine and immune cells.

The oncolytic viruses are a type of tumor-killing viruses with replication ability and now are accepted by the public as an important branch of tumor immunotherapy. Oncolytic viruses can specifically target and infect tumor cells, for example, by taking advantage of the inactivation or defects of tumor suppressor genes in tumor cells to selectively infect tumor cells. After oncolytic viruses infect tumor cells, they will replicate in large quantities in tumor cells and eventually destroy tumor cells, thereby killing tumor cells. Moreover, oncolytic viruses can also provide immune stimulation signals necessary to enhance the host's own anti-cancer response, thereby attracting more immune cells to continue to kill residual tumor cells. Therefore, oncolytic viruses have the ability to break the microenvironment of tumor tissues and turn "cold tumors" into "hot tumors".

Moreover, an oncolytic virus vaccine is constructed by an oncolytic virus expressing tumor-associated antigens in tumor cells. The oncolytic virus vaccine can not only use the oncolytic virus itself to kill tumor cells and break the microenvironment of tumor tissue, but also use tumor-associated antigens expressed by the oncolytic virus to target tumor cells (MARK), transforming "tumors that have no tumor-associated antigen target and cannot be treated" into "tumors that have tumor-associated antigen targets and can be treated". An oncolytic virus vaccine and immune cells targeting the tumor-associated antigen target are used in combination to attack and kill tumor cells, where the tumor antigen expressed by the oncolytic virus vaccine can guide the immune cells to reach the target tumor tissue center, and the oncolytic virus further can work together with the immune cells to kill tumor cells, achieving a curative effect where 1+1 is greater than 2.

Further, in order to improve the curative effect, cytokines may further be inserted into the oncolytic virus. Under the synergistic effect of three anti-tumor mechanisms: oncolytic virus, cytokine and immune cells, a greater anti-tumor effect is achieved.

The present application provides a method for treating tumors using a combination of an oncolytic virus vaccine and immune cells, adopting the following technical solutions:

A method for treating tumors using a combination of an oncolytic virus vaccine and an immune cell wherein the oncolytic virus vaccine and immune cells are used in combination for treating the tumors; the oncolytic virus vaccine includes a recombinant oncolytic virus expressing a tumor antigen and is used for targeting tumor cells; the immune cells express a chimeric antigen receptor paired with the tumor antigen, and are used for killing or destroying the tumor cells targeted.

The recombinant oncolytic virus includes an M protein, a G protein, an N protein, a P protein, and an L protein.

Compared to an amino acid sequence shown in SEQ ID NO 1, the M protein includes site mutations of M51R, V221F, and S226R; or the M protein includes site mutations of N32S, N49D, M51R, H54Y, V221F, V225I, and S226R; or the M protein includes site mutations of N32S, N49D, M51R, H54Y, knockouting of bases encoding leucine at position 111, V221F, V225I, and S226R; or the M protein includes site mutations of N32S, N49D, M51R, H54Y, L111A, V221F, V225I, and S226R; or the M protein includes site mutations of G21E, N32S, N49D, M51R, H54Y, V221F, V225I, and S226R; or the M protein includes site mutations of G21E, N32S, M33A, N49D, M51R, H54Y, V221F, V225I, and S226R; or the M protein includes site mutations of G21E, N32S, M33A, N49D, M51R, H54Y, A133T, V221F, V225I, and S226R; or the M protein includes site mutations of N32S, M33A, N49D, M51R, H54Y, V221F, V225I, and S226R; or the M protein includes site mutations of N32S, M33A, N49D, M51R, H54Y, A133T, V221F, V225I, and S226R; or the M protein includes site mutations of N32S, N49D, M51R, H54Y, A133T, V221F, V225I, and S226R.

Compared to an amino acid sequence shown in SEQ ID NO 12, the G protein includes site mutations of V53I, A141V, D172Y, K217E, D232G, V331A, V371E, G436D, T438S, F453L, T471I, and Y487H.

Compared to an amino acid sequence shown in SEQ ID NO 14, the N protein includes site mutations of I14V, R155K, and S353N.

Compared to an amino acid sequence shown in SEQ ID NO 16, the P protein includes site mutations of R50K, V76A, D99E, L126S, L140S, H151Y, I168M, K170E, Y189S, and N237D.

Compared to an amino acid sequence shown in SEQ ID NO 18, the L protein includes site mutations of S87P and I487T.

In the present application, an M protein in a wild-type Indiana MuddSummer subtype strain of VSV includes an amino acid sequence as shown in SEQ ID NO 1.

In a specific embodiment, the M protein includes an amino acid sequence as shown in SEQ ID NO 2.

In a specific embodiment, the M protein includes an amino acid sequence as shown in SEQ ID NO 3.

In a specific embodiment, the M protein includes an amino acid sequence as shown in SEQ ID NO 4.

In a specific embodiment, the M protein includes an amino acid sequence as shown in SEQ ID NO 5.

In a specific embodiment, the M protein includes an amino acid sequence as shown in SEQ ID NO 6.

In a specific embodiment, the M protein includes an amino acid sequence as shown in SEQ ID NO 7.

In a specific embodiment, the M protein includes an amino acid sequence as shown in SEQ ID NO 8.

In a specific embodiment, the M protein includes an amino acid sequence as shown in SEQ ID NO 9.

In a specific embodiment, the M protein includes an amino acid sequence as shown in SEQ ID NO 10.

In a specific embodiment, the M protein includes an amino acid sequence as shown in SEQ ID NO 11.

In the present application, a G protein in the wild-type Indiana MuddSummer subtype strain of the VSV includes an amino acid sequence as shown in SEQ ID NO 12.

In a specific embodiment, the G protein includes an amino acid sequence as shown in SEQ ID NO 13.

In the present application, an N protein in the wild-type Indiana MuddSummer subtype strain of the VSV includes an amino acid sequence as shown in SEQ ID NO 14.

In a specific embodiment, the N protein includes an amino acid sequence as shown in SEQ ID NO 15.

In the present application, a P protein in the wild-type Indiana MuddSummer subtype strain of the VSV includes an amino acid sequence as shown in SEQ ID NO 16.

In a specific embodiment, the N protein includes an amino acid sequence as shown in SEQ ID NO 17.

In the present application, an L protein in the wild-type Indiana MuddSummer subtype strain of the VSV includes an amino acid sequence as shown in SEQ ID NO 18.

In a specific embodiment, the L protein includes an amino acid sequence as shown in SEQ ID NO 19.

In some specific embodiments, the recombinant oncolytic virus is obtained by carrying out site-directed mutagenesis on a rhabdovirus.

In some specific embodiments, the recombinant oncolytic virus is obtained by carrying out site-directed mutagenesis on a vesicular stomatitis virus (VSV).

In some specific embodiments, the recombinant oncolytic virus is obtained by carrying out site-directed mutagenesis on an Indiana MuddSummer subtype strain of the VSV.

Further, the tumor antigen is selected from a solid tumor antigen or a hematological tumor antigen.

Further, the solid tumor antigen includes but is not limited to 5T4, RORI, EGFR, FcγRI (CD64), FcγRIIa (CD32a), FcγRIIb (CD32b), CD28, CD137 (4-1BB), CTLA-4, HER-2, FAS, FAP (fibroblast activation protein), LGR5, C5aR1, A2AR, fibroblast growth factor receptor 1 (FGFR1), FGFR2, FGFR3, FGFR4, glucocorticoid-induced TNFR-related (GITR) protein, lymphotoxin-β receptor (LTβR), tumor necrosis factor-related apoptosis-inducing ligand receptor 1 (TRAIL receptor 1), TRAIL receptor 2, prostate-specific membrane antigen (PSMA) protein, prostate stem cell antigen (PSCA) protein, tumor-related protein carbonic anhydrase IX (CAIX), epidermal growth factor receptor 1 (EGFR1), EGFRvIII, ErbB3 (HER3), folate receptor, ephrin receptor, PDGFRa, ErbB-2, CD2, CD40, CD74, CD80, CD86, CCAM5 (CD66e), CCAM6 (CD66c), p53, cMet (tyrosine protein kinase Met), HGFR, MAGE-A1, MAGE-A2, MAGE-A3, MAGE-A4, MAGE-A6, MAGE-A10, MAGE-A12, BACE, DAM-6, DAM-10, GAGE-1, GAGE-2, GAGE-8, GAGE-3, GAGE-4, GAGE-5, GAGE-6, GAGE-7B, NA88-A, NY-ESO-1, BRCA1, BRCA2, MART-1, MCIR, Gp100, PSA, PSM, tyrosinase, TRP-1, TRP-2, ART-4, CAMEL, Cyp-B, hTERT, hTRT, iCE, MUC2, P-cadherin, myostatin (GDF8), Cripto (TDGF1), MUC5AC, PRAME, P15, RU1, RU2, SART-1, SART-3, AFP, β-catenin/m, caspase-8/m, CDK-4/m, ELF2M, GnT-V, G250, HSP70-2M, HST-2, KIAA0205, MUM-1, MUM-2, MUM-3, myosin/m, RAGE, SART-2, TRP-2/INT2, 707-AP, annexin II, CDC27/m, TPI/mbcr-abl, ETV6/AML, LDLR/FUT, Pml/RARa, TEL/AML1, CD28, CD137, CanAg, mesothelin (MSLN), DR5, PD-1, PD-L1, IGF-1R, CXCR4, neuropilin 1 (NRP-1), glypican2/3 (GPC2/3), EphA2, B7-H3, B7-H4, gpA33, GPC3, SSTR2, GD2, VEGF-A, VEGFR-2, PDGFR-a, ANKL, RANKL, MSLN, EBV, TROP2, FOLR1, AXL, Claude 18.2, MUC1, and TPBG.

Further, the hematological tumor antigen includes but is not limited to BCMA (TNFRSF17), CD4, CD5 (Leu-1), CD7, CD10, FcγRIIIa (CD16a), FcγRIIIb (CD16b), CD19, CD20 (MS4A1), CD22 (Siglec-2), CD23, CD30 (TNFRSF8), CD33 (Siglec-3), CD34, CD37, CD38, CD44, CD47, CD56 (NCAM1), CD70, CD117, CD123 (IL3RA), CD138 (SDC1), CD174, CLL-1, ROR1, NKG2DL1/2 (ULBP1/2), IL1R3 (IL-1-RAP), FCRL5, GPRC5D, CLEC12A, WT1, FLT3, TLR8, SHP2, KAT6A/B, CSNK1A1, FLI1, IKZF1/3, PI3K, c-Kit, SLAMF3 (CD229), SLAMF7 (CD319), TCRB-chain, ITGB7, k-1gG, TACI, TRBCI, and LeY.

Further, the tumor antigen is one or more selected from a group consisting of: CD19, CD22, BCMA, MUC1, NY-ESO-1, MAGE A4, cMet, Claude 18.2, MSLN, EGFR, VEGFR2, HER-2, TPBG, AFP, and MAGE-A10.

In a specific embodiment, the tumor antigen is CD19.

In a specific embodiment, the tumor antigen is CD22.

In a specific embodiment, the tumor antigen is BCMA.

In a specific embodiment, the tumor antigen is MUC-1.

In a specific embodiment, the tumor antigen is NY-ESO-1.

In a specific embodiment, the tumor antigen is MAGE A4.

In a specific embodiment, the tumor antigen is cMet.

In a specific embodiment, the tumor antigen is Claude 18.2.

In a specific embodiment, the tumor antigen is MSLN.

In a specific embodiment, the tumor antigen is EGFR.

In a specific embodiment, the tumor antigen is VEGFR2.

In a specific embodiment, the tumor antigen is HER-2.

In a specific embodiment, the tumor antigen CD19 includes an amino acid sequence as shown in SEQ ID NO 20.

In a specific embodiment, the tumor antigen CD22 includes an amino acid sequence as shown in SEQ ID NO 21.

In a specific embodiment, the tumor antigen BCMA includes an amino acid sequence as shown in SEQ ID NO 22.

In a specific embodiment, the tumor antigen MUC-1 includes an amino acid sequence as shown in SEQ ID NO 23.

In a specific embodiment, the tumor antigen NY-ESO-1 includes an amino acid sequence as shown in SEQ ID NO 24.

In a specific embodiment, the tumor antigen MAGE A4 includes an amino acid sequence as shown in SEQ ID NO 25.

In a specific embodiment, the tumor antigen cMet includes an amino acid sequence as shown in SEQ ID NO 26.

In a specific embodiment, the tumor antigen Claude 18.2 includes an amino acid sequence as shown in SEQ ID NO 27.

In a specific embodiment, the tumor antigen MSLN includes an amino acid sequence as shown in SEQ ID NO 28.

In a specific embodiment, the tumor antigen EGFR includes an amino acid sequence as shown in SEQ ID NO 29.

In a specific embodiment, the tumor antigen VEGFR2 includes an amino acid sequence as shown in SEQ ID NO 30.

In a specific embodiment, the tumor antigen HER-2 includes an amino acid sequence as shown in SEQ ID NO 31.

In a specific embodiment, the tumor antigen TPBG includes an amino acid sequence as shown in SEQ ID NO 32.

Further, the recombinant oncolytic virus expresses at least one or more of the tumor antigen.

Further, the immune cells are selected from T cells, NK cells and M cells.

Further, the immune cells are selected from CAR-T cells, TCR-T cells, CAR-γδ-T cells, CAR-Crispered-T cells, STAR-T cells, CAR-NK cells, and CAR-M cells.

Further, the immune cells are selected from autologous cells, allogeneic cells, or immune cells derived from IPSC.

Further, the immune cells include immune cells obtained by means of in vitro expansion.

Further, the immune cells express at least one or more the antigen receptor.

Further, the recombinant oncolytic virus further includes a cytokine encoded by an exogenous gene.

Further, the cytokine is selected from interleukins (IL), interferons (IFN), tumor necrosis factors (TNF), colony stimulating factors (CSF), transforming growth factor-β family (TGF-β family), and chemokine family.

Further, the cytokine is one or more selected from a group consisting of: GM-CSF, G-CSF, M-CSF, IL-1, IL-2, IL-4, IL-5, IL-6, IL-9, IL-10, IL-12, IL-13, IL-15, IL-17, IL-18, IL-23, IL-27, IFN-a, IFN-β, IFN-γ, IFN-β, TGF-β, and TNF-α.

Further, the cytokine is one or more selected from a group consisting of: GM-CSF, IL-2, IL-12, IL-15, IL-18, TNF-$\alpha$, and IFN-$\beta$.

In a specific embodiment, the cytokine is GM-CSF.

In a specific embodiment, the cytokine is IL-2.

In a specific embodiment, the cytokine is IL-12.

In a specific embodiment, the cytokine is IL-15.

In a specific embodiment, the cytokine is IL-18.

In a specific embodiment, the cytokine is TNF-$\alpha$.

In a specific embodiment, the cytokine is IFN-$\beta$.

In a specific embodiment, the cytokine IL-12 includes an amino acid sequence as shown in SEQ ID NO 33.

In a specific embodiment, the cytokine IL-12 includes an amino acid sequence as shown in SEQ ID NO 34.

In a specific embodiment, the cytokine IL-15 includes an amino acid sequence as shown in SEQ ID NO 35.

In a specific embodiment, the cytokine IL-18 includes an amino acid sequence as shown in SEQ ID NO 36.

In some specific embodiments, the recombinant oncolytic virus includes a nucleic acid molecule; the nucleic acid molecule includes a nucleic acid sequence encoding the M protein with the site mutations, a nucleic acid sequence encoding the G protein with the site mutations, a nucleic acid sequence encoding the N protein with the site mutations, a nucleic acid sequence encoding the P protein with the site mutations, a nucleic acid sequence encoding the L protein with the site mutations, a nucleic acid sequence encoding the tumor antigen, and a nucleic acid sequence encoding the cytokine.

In a specific embodiment, in the nucleic acid molecule, the nucleic acid sequence encoding the tumor antigen is located between the nucleic acid sequence encoding the G protein with the site mutations and the nucleic acid sequence encoding the L protein with the site mutations.

In a specific embodiment, in the nucleic acid molecule, the nucleic acid sequence encoding the cytokine is located between the nucleic acid sequence encoding the G protein with the site mutations and the nucleic acid sequence encoding the L protein with the site mutations.

In a specific embodiment, in the nucleic acid molecule, the nucleic acid sequence encoding the cytokine is located between the nucleic acid sequence encoding the tumor antigen and the nucleic acid sequence encoding the L protein with the site mutations, or between the nucleic acid sequence encoding the G protein with the site mutations and the nucleic acid sequence encoding the tumor antigen.

In some specific embodiments, the method for treating tumors using a combination of an oncolytic virus vaccine and immune cells is used for killing an abnormally proliferative cell in a sustained manner.

In some specific embodiments, the abnormally proliferative cell is selected from tumor cells or tumor tissue-related cells.

In some specific embodiments, the tumors include solid tumors or hematological tumors.

In some specific embodiments, the tumors include but are not limited to acute lymphoblastic leukemia, acute B-cell leukemia, chronic non-lymphocytic leukemia, non-Hodgkin's lymphoma, anal cancer, astrocytoma, basal cell carcinoma, bile duct cancer, bladder cancer, mastocarcinoma, breast cancer, cervical cancer, chronic myeloproliferative tumors, colorectal cancer, endometrial cancer, ependymoma, esophageal cancer, diffuse large B-cell lymphoma (DLBCL), sensorineuroblastoma, Ewing's sarcoma, fallopian tube cancer, gallbladder cancer, gastric cancer, gastrointestinal carcinoid tumors, hepatocellular carcinoma, hypopharyngeal cancer, Kaposi sarcoma, kidney cancer, Langerhans cell hyperplasia, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumors, pharyngeal cancer, pituitary tumors, prostate cancer, rectal cancer, renal cell carcinoma, retinoblastoma, skin cancer, small cell lung cancer, small intestine cancer, squamous neck cancer, testicular cancer, thymoma, thyroid cancer, uterine cancer, vaginal cancer and vascular tumors.

The present application further provides a composition; the composition includes the oncolytic virus vaccine and the immune cells.

In summary, the present application has the following beneficial effects:

The method for treating tumors using a combination of an oncolytic virus vaccine and immune cells provided by the present application has a better killing ability to abnormally proliferative (tumor) LLC cells in vitro, and a relatively poor killing ability to normal MEF cells in vitro. In view of this, the method for treating tumors using a combination of an oncolytic virus vaccine and immune cells provided by the present application can be well used for infecting and killing tumor cells, cancer cells and other cells, and are not easily eliminated in tumor cells, cancer cells and other cells, further improving the cure rate of the recombinant oncolytic virus for tumor cells, cancer cells and other cells. Moreover, the method for treating tumors using a combination of an oncolytic virus vaccine and immune cells described above will not cause damages to normal cells and the recombinant oncolytic virus can be more easily eliminated in normal cells, thereby further ensuring the safety of normal cells.

## BRIEF DESCRIPTION OF DRAWINGS

FIG. 1 shows test results of the killing ability of the method for treating tumors using a combination of the oncolytic virus vaccines prepared in Preparation Examples 9-83 of the present application and immune cells to LLC cells in vitro and the killing ability of a wild-type oncolytic virus to LLC cells in vitro.

FIG. 2 shows test results of the killing ability of the method for treating tumors using a combination of the oncolytic virus vaccines prepared in Preparation Examples 9-83 of the present application and immune cells to MEF cells in vitro and the killing ability of a wild-type oncolytic virus to MEF cells in vitro.

FIG. 3 shows test results of the killing ability of the oncolytic virus vaccines prepared in Preparation Examples 84-95 of the present application, the immune cells prepared in Preparation Examples 96-113 of the present application, and a wild-type oncolytic virus to LLC cellsin vitro.

FIG. 4 shows test results of the killing ability of the oncolytic virus vaccines prepared in Preparation Examples 84-95 of the present application, the immune cells prepared in Preparation Examples 96-113 of the present application, and a wild-type oncolytic virus to MEF cells in vitro.

FIG. 5 shows tumor cell killing rates of the method for treating tumors using a combination of the oncolytic virus vaccines prepared in Preparation Examples 9-83 of the present application and immune cells.

FIG. 6 shows tumor cell killing rates of the oncolytic virus vaccines prepared in Preparation Examples 84-95 of the present application and the immune cells prepared in Preparation Examples 96-113 of the present application.

In the above figures, number 0 on the horizontal axis represents the wild-type oncolytic virus; numbers 9-113 on the horizontal axis represent Preparation Examples 9-113, respectively.

$OD_{570}$ on the vertical axis represents the OD value of the cell. The larger the value of $OD_{570}$, the poorer the killing ability of a recombinant oncolytic virus to the cell; the smaller the value of $OD_{570}$, the better the killing ability of a recombinant oncolytic virus to the cell.

Those skilled in the art can easily perceive other aspects and advantages of the present application from the following detailed description. Only exemplary embodiments of the present application are shown and described in the following detailed description. As those skilled in the art will appreciate, the teachings of the present application enable those skilled in the art to make modifications to the specific embodiments disclosed without departing from the spirit and scope of the invention to which the present application relates. Accordingly, the drawings and descriptions in the specification of the present application are merely illustrative and not restrictive.

## DESCRIPTION OF EMBODIMENTS

The implementation of the present application will be described below by means of specific embodiments. Those skilled in the art can easily understand other advantages and effects of the present application from the contents described herein.

## DEFINITIONS

The term "oncolytic virus", as used herein, generally refers to a virus that can replicate in tumor cells and kill tumor cells. Oncolytic viruses include, but are not limited to, vesicular stomatitis virus (VSV), poxvirus, herpes simplex virus (HSV), measles virus, Semliki Forest virus, poliovirus, reovirus, Seneca Valley virus (SVV), Echo-type enterovirus, coxsackievirus, Newcastle disease virus (NDV), and Maraba virus. In certain embodiments, the oncolytic virus is modified to improve the selectivity for tumor cells. In certain embodiments, the oncolytic virus is modified to reduce its immunogenicity.

In some embodiments, the VSV is a mutant of the Indiana MuddSummer subtype strain of the VSV and can be used to treat a tumor. This virus does not interact with endogenous IFN-β in normal cells and can only selectively amplify and grow in tumor cells.

The VSV can express a variety of cell surface molecules, including low-density lipoprotein receptors, phosphatidylserine, sialolipids, and heparan sulfate, and can attach to the cell surface by these molecules. Compared with other oncolytic virus platforms currently under development, the VSV has the following advantages: (1) small genome, short replication time, and fast transsynaptic speed; (2) extremely high expression of exogenous genes, resulting in high titers and a possibility of large-scale production; and (3) independent cell cycle and no risk of transformation in the cytoplasm of host cells. This oncolytic virus will not integrate into DNA, and after being attenuated, this oncolytic virus can avoid the nervous system inflammation caused by wild-type viruses. In view of the above characteristics, the VSV has great potential in tumor immunotherapy.

In some embodiments, the M protein, and/or the G protein, and/or the N protein, and/or the P protein, and/or the L protein of the VSV may be subjected to site-directed mutagenesis.

In certain embodiments, the recombinant oncolytic virus described herein may be an oncolytic virus that has been genetically modified, such as modified by modification of one or more genes, so as to improve its tumor selectivity and/or preferentially replicate in dividing cells. The "genetically modified" may refer to modification of genes involved in DNA/RNA replication, nucleic acid metabolism, host tropism, surface attachment, virulence, lysis and diffusion, or modification of integrating exogenous genes. The exogenous genes may include exogenous immune regulatory genes, exogenous screening genes and exogenous reporter genes. The oncolytic virus that has been modified may also be an oncolytic virus modified at an amino acid level, such as, by insertion, deletion, or substitution of one or more amino acids.

The term "M protein", as used herein, generally refers to a matrix protein of the VSV. The M protein is an important virulence factor of the VSV and is also a protein in the VSV that is known to interfere with the natural immune response of mice. The term "M protein" also encompasses homologs, orthologs, variants and functionally active fragments thereof. In the present application, the M protein in a wild-type Indiana MuddSummer subtype strain of the VSV may include an amino acid sequence as shown in SEQ ID NO 1. In the present application, the M protein of the oncolytic virus may include amino acid sequences as shown in SEQ ID NOs 2-11.

The term "G protein", as used herein, generally refers to a glycoprotein of the VSV, also known as the envelope protein. The term "G protein" also encompasses homologs, orthologs, variants and functionally active fragments thereof. In the present application, the G protein in a wild-type Indiana MuddSummer subtype strain of the VSV may include an amino acid sequence as shown in SEQ ID NO 12. In the present application, the G protein of the oncolytic virus may include an amino acid sequence as shown in SEQ ID NO 13.

The term "N protein", as used herein, generally refers to a nucleocapsid protein of the VSV. The term "N protein" also encompasses homologs, orthologs, variants and functionally active fragments thereof. In the present application, the N protein in Indiana MuddSummer subtype of a wild-type VSV may include an amino acid sequence as shown in SEQ ID NO 14. In the present application, the N protein of the oncolytic virus may include an amino acid sequence as shown in SEQ ID NO 15.

The term "P protein", as used herein, generally refers to a phosphoprotein of the VSV. The term "P protein" also encompasses homologs, orthologs, variants and functionally active fragments thereof. In the present application, the P protein in a wild-type Indiana MuddSummer subtype strain of the VSV may include an amino acid sequence as shown in SEQ ID NO 16. In the present application, the P protein of the oncolytic virus may include an amino acid sequence as shown in SEQ ID NO 17.

The term "L protein", as used herein, generally refers to an RNA poly E protein of the VSV. An L gene of the VSV encodes the RNA poly E protein. The term "L protein" also encompasses homologs, orthologs, variants and functionally active fragments thereof. In the present application, the L protein in a wild-type Indiana MuddSummer subtype strain of the VSV may include an amino acid sequence as shown in SEQ ID NO 18. In the present application, the L protein of the oncolytic virus may include an amino acid sequence as shown in SEQ ID NO 19.

In the present application, the mutated site of a protein is generally expressed as "amino acid + amino acid position + amino acid after mutation". In the present application, the mutation may include but is not limited to the addition, substitution absence and/or deletion of an amino acid. For example, the term "M51R" generally refers to the mutation of methionine (M) at position 51 to arginine (R).

The term "amino acid substitution", as used herein, generally refers to the substitution of an amino acid residue present in a parent sequence with another amino acid residue. The amino acid in the parent sequence may be substituted, for example, via chemical synthesis or by recombinant methods known in the art. Therefore, "substitution at position xx" generally refers to the substitution of an amino acid present at position xx with an alternative amino acid residue. In the present application, the amino acid substitution may include an amino acid mutation.

In the present application, the term "mutation" generally refers to a change in the nucleotide or amino acid sequence of a wild-type molecule. Amino acid changes may include the substitution, deletion, absence, insertion, addition or truncation of an amino acid, or the processing or cleavage of a protein.

In the present application, the recombinant oncolytic virus means integration of an exogenous gene at the same time of a site-directed mutagenesis on the M protein, and/or G protein, and/or N protein, and/or P protein, and/or L protein of the VSV. The exogenous gene specifically refers to a gene encoding a tumor antigen and/or a cytokine.

In this application, the term "antigen" refers to a substance that can cause the production of antibodies or immune cells, and is any substance that can induce an immune response in the body. That is, the antigen is a substance that can be specifically recognized and bound by the antigen receptor (TCR/BCR) on the surface of T/B lymphocytes, activate T/B cells, cause them to proliferate and differentiate, produce immune response products (sensitized lymphocytes or antibodies), and can specifically bind to the corresponding products in vivo and in vitro. Therefore, antigenic substances have two important characteristics: immunogenicity and immunoreactivity. Immunogenicity refers to the ability of antigens to induce specific immune responses in the body and produce antibodies and/or sensitized lymphocytes. Immunoreactivity refers to the ability to specifically bind to corresponding immune effector substances (antibodies or sensitized lymphocytes) in vivo and in vitro.

In a specific embodiment, the oncolytic virus is engineered to carry a coding sequence for an antigen that can be recognized by CAR-T cells.

In some specific embodiments, the antigen is exogenous, meaning that the antigen comes from a different species.

In some specific embodiments, the antigen is an endogenous antigen. Specifically, the antigen is an antigen that is usually expressed on tumor cells.

In a specific embodiment, the antigen is a tumor associated antigen (TAA) or a tumor specific antigen (TSA).

In a specific embodiment, the TAA or TSA encompasses molecules or portions thereof that are presented on the cell surface (antigens recognized by CARs) or within the cell membrane (antigens recognized by TCRs) or present in the tumor environment (e.g., in the tumor microenvironment).

In some specific embodiments, the cell is a tumor cell.

In some specific embodiments, the TAA or TSA includes a tumor associated antigen or a tumor specific antigen on the cell surface or in the cell membrane.

In some specific embodiments, the cell is a non-tumor cell present in a tumor environment. The cell is, for example, but not limited to, a cell present in the vasculature tissue associated with a tumor or cancer.

In some specific embodiments, the TAA or TSA is an angiogenic antigen in a tumor microenvironment.

In some specific embodiments, the TAA or TSA is an antigen on a blood vessel in a tumor microenvironment.

In some specific embodiments, the cell is a stromal cell present in a tumor environment.

In some specific embodiments, the TAA or TSA is a stromal cell antigen in a tumor microenvironment.

In some specific embodiments, the TAA or TSA includes an extracellular epitope of a tumor cell surface antigen, a tetramer inside or outside the tumor cell membrane, or other structures that can be recognized by antibodies or immune cells.

In some specific embodiments, the TAA or TSA includes an extracellular matrix antigen.

In some specific embodiments, the TAA or TSA includes an antigen present in a tumor microenvironment (TME).

In some specific embodiments, the TAA or TSA includes a molecule secreted by a tumor cell into a TME.

In some specific embodiments, the TAA or TSA includes an effector molecule secreted by a tumor cell into a TME.

In some specific embodiments, the TAA or TSA includes an effector molecule secreted by a tumor cell into a TME to downregulate or inhibit the activity of cytotoxic natural killer (NK) cells or T cells.

In some specific embodiments, the TAA or TSA includes a soluble activating receptor ligand that is secreted by a tumor cell into a TME to block recognition of tumor cells by NK cells or T cells.

In some specific embodiments, examples of TAA or TSA include but are not limited to 5T4, RORI, EGFR, FcγRI, FcγRIIa, FcγRIIb, FcγRIIIa, FcγRIIIb, CD28, CD137, CTLA-4, FAS, FAP (fibroblast activation protein), LGR5, C5aR1, A2AR, fibroblast growth factor receptor 1 (FGFR1), FGFR2, FGFR3, FGFR4, glucocorticoid-induced TNFR-related (GITR) protein, lymphotoxin-β receptor (LTβR), toll-like receptor (TLR), tumor necrosis factor-related apoptosis-inducing ligand receptor 1 (TRAIL receptor 1), TRAIL receptor 2, prostate-specific membrane antigen (PSMA) protein, prostate stem cell antigen (PSCA) protein, tumor-related protein carbonic anhydrase IX (CAIX), epidermal growth factor receptor 1 (EGFR1), EGFRvIII, human epidermal growth factor receptor 2 (Her2/neu; Erb2), ErbB3 (Her3), folate receptor, ephrin receptor, PDGFRa, ErbB2, CD2, CD20, CD22, CD30, CD33, CD40, CD37, CD38, CD70, CD74, CD56, CD80, CD86, CD123, CCAM5, CCAM6, BCMA, p53, cMet (tyrosine protein kinase Met), hepatocyte growth factor receptor (HGFR), MAGE-A1, MAGE-A2, MAGE-A3, MAGE-A4, MAGE-A6, MAGE-A10, MAGE-A12, BAGE, DAM-6, DAM-10, GAGE-1, GAGE-2, GAGE-8, GAGE-3, GAGE-4, GAGE-5, GAGE-6, GAGE-7B, NA88-A, NY-ESO-1, BRCAI, BRCA2, MART-1, MCIR, Gp100, PSA, PSM, tyrosinase, Wilms tumor antigen (WT1), TRP-1, TRP-2, ART-4, CAMEL, Cyp-B, hTERT, hTRT, iCE, MUC1, MUC2, P-cadherin, myostatin (GDF8), Cripto(TDGF1), MUC5AC, PRAME, P15, RU1, RU2, SART-1, SART-3, WT1, AFP, β-catenin/m, caspase-8/m, CDK-4/m, ELF2M, GnT-V, G250, HSP70-2M, HST-2, KIAA0205, MUM-1, MUM-2, MUM-3, myosin/m, RAGE, SART-2, TRP-2/INT2, 707-AP, annexin II, CDC27/m, TPI/mbcr-abl, ETV6/AML, LDLR/FUT, Pml/RARa, TEL/AML1, CD28, CD137, CanAg, mesothelin, DR5, PD-1, PD-L1, HER-2, IGF-1R, CXCR4, neuropilin 1, glypicans, EphA2, CD138, B7-H3, B7-H4, gpA33, GPC3, SSTR2 or VEGF-R2.

Exemplary immune cells that can be used herein include, but are not limited to, dendritic cells (including immature and mature dendritic cells), T lymphocytes (such as naive T cells, effector T cells, memory T cells, cytotoxic T lymphocytes, T helper cells, natural killer T cells, Treg cells, tumor infiltrating lymphocytes (TIL) and lymphokine-activated killer (LAK) cells), B cells, natural killer (NK) cells, NKT cells, αβT cells, γδT cells, monocytes, macrophages, neutrophils, granulocytes, peripheral blood mononuclear cells (PBMC) and combinations thereof. Immune cell subsets can be defined by the presence or absence of one or more cell surface markers (such as CD3, CD4, CD8, CD19, CD20, CD11c, CD123, CD56, CD34, CD14, and CD33) known in the art. In cases where the pharmaceutical composition includes multiple engineered mammalian immune cells, these engineered mammalian immune cells may be specific subsets of immune cell types, combinations of subsets of immune cell types, or combinations of two or more immune cell types.

NK cells are innate lymphocytes, the third major type of lymphocytes in addition to T cells and B cells. The NK cells non-specifically kill tumor cells without antigen pre-sensitization.

In adoptive NK cell therapy patients are infused with healthy and cytokine-activated NK cells. NK cells are collected by different channels, such as peripheral blood NK cells, NK cells from cord blood or placenta, and NK cells differentiated from induced pluripotent stem cells (IPSC), and infused after enhancing their functions in vitro, thereby enhancing the tumor-killing ability of patients.

Genetically engineered NK cell therapy is different from adoptive NK cell therapy in that this therapy uses the infusion of genetically modified NK cells, including CAR-NK cells, to achieve targeted killing and enhance its anti-tumor effect. Compared with CAR-T therapy, CAR-NK cells have advantages such as a reduced risk of cytokine release syndrome. Based on the success of CAR-T, the popularity of CAR-NK as a treatment method is increasing year by year. Relevant researchers have developed innovative responses to challenges faced by the CAR-NK therapy such as difficulty in vitro expansion, low transfection efficiency, and poor cell persistence.

In some specific embodiments, the immune cells are present in a homogeneous population of cells. In some specific embodiments, the immune cells are present in a heterogeneous population of cells that can be enhanced in immune cells. In some specific embodiments, the immune cells are lymphocytes. In some specific embodiments, the immune cells are not lymphocytes. In some specific embodiments, the immune cells are suitable for adoptive immunotherapy. In some specific embodiments, the immune cell are PBMCs. In some embodiments, the engineered immune cells are derived from immune cells of PBMC. In some specific embodiments, the immune cells are T cells. In some specific embodiments, the immune cells are CD4 +T cells. In some specific embodiments, the immune cells are CD8+ T cells. In some specific embodiments, the immune cells are T cells that express TCR $\alpha$ and TCR $\beta$ chains (i.e., $\alpha\beta$T cells). In some specific embodiments, the immune cells are T cells that express TCR$\gamma$ and TCR$\delta$ chains (i.e., $\gamma\delta$T cells). In some specific embodiments, the immune cells are $\gamma9\delta2$ T cells. In some specific embodiments, the immune cells are $\delta1$ T cells. In some specific embodiments, the immune cells are $\delta3$ T cells.

In some specific embodiments, the immune cells are B cells. In some specific embodiments, the immune cells are NK cells. In some specific embodiments, the immune cells are NK-T cells. In some specific embodiments, the immune cells are dendritic cells (DC). In some specific embodiments, the immune cells are DC-activated T cells.

In some specific embodiments, the immune cells are derived from primary cells. In some specific embodiments, the immune cells are primary cells isolated from an individual. In specific embodiments, the immune cells are propagated (such as, proliferated and/or differentiated) from primary cells isolated from an individual. In some specific embodiments, the primary cells are obtained from the thymus. In some specific embodiments, the primary cells are obtained from a lymph or lymph node (such as a tumor-draining lymph node). In some specific embodiments, the primary cells are obtained from the spleen. In some specific embodiments, the primary cells are obtained from bone marrow. In some specific embodiments, the primary cells are obtained from blood, such as peripheral blood. In some specific embodiments, the primary cells are peripheral blood mononuclear cells (PBMC). In some specific embodiments, the primary cells are derived from plasma. In some specific embodiments, the primary cells are derived from tumors. In some specific embodiments, the primary cells are obtained from the mucosal immune system. In some specific embodiments, the primary cells are obtained from a biopsy sample.

In some specific embodiments, the immune cells are derived from a cell line. In some specific embodiments, the immune cells are obtained from commercial cell lines. In specific embodiments, the immune cells are propagated (such as, proliferated and/or differentiated) from a cell line established by primary cells isolated from an individual. In some specific embodiments, the cell line is a mortal cell line. In some specific embodiments, the cell line is an immortalized cell line. In some specific embodiments, the cell line is a tumor cell line, such as a leukemia or lymphoma cell line. In some specific embodiments, the cell line is a cell line derived from PBMC. In some specific embodiments, the cell line is a stem cell line. In some specific embodiments, the cell line is NK-92. In some specific embodiments, the engineered immune cells are derived from stem cells. In some specific embodiments, the stem cells are embryonic stem cells (ESC). In some specific embodiments, the stem cells are hematopoietic stem cells (HSC). In some specific embodiments, the stem cells are mesenchymal stem cells. In some embodiments, the stem cells are induced pluripotent stem cells (IPSC).

The term "cytokines", as used herein, refers to biologically active substances synthesized and secreted by immune cells (such as lymphocytes, monocytes and macrophages) and their related cells (such as vascular endothelial cells and fibroblasts) to regulate the functions of other immune cells or target cells. The cytokines belong to small molecule polypeptides or glycoproteins. Cytokines with immune regulation effects can be expressed by recombinant oncolytic viruses. According to their main functions, cytokines are divided into: interleukins (IL), interferons (IFN), tumor necrosis factors (TNF), colony stimulating factors (CSF), transforming growth factor-$\beta$ family (TGF-$\beta$ family), growth factors (GF), and chemokine family.

Interleukins include IL-1, IL-2, IL-7, IL-9, IL-15, IL-21, IL-4, IL-12 and IL-18.

Specifically, interleukin 1 (IL-1): IL-1 is a pleiotropic cytokine involved in cortical inflammatory response, cell growth and tissue repair. The IL-1 superfamily has 11 members, such as IL-1A, IL-1B, IL-1Ra, and IL-18. IL-1 is a drug target for some cancers and is also used in cell therapy. In terms of cellular immunotherapy, IL-1 can stimulate the proliferation of CD4+ T cells in vitro, induce the production of IL-2, co-stimulate the activation of CD8+/IL1R+ T cells, and stimulate the proliferation of mature B cell and the secretion of immunoglobulin.

Specifically, interleukin 2 (IL-2): IL-2, also known as T cell growth factor, is produced by T cells in response to antigens or mitotic stimulation and is widely used to promote the activation and proliferation of T cells and NK cells. IL-2 can stimulate proliferation of NK cells, increase cytotoxicity, and stimulate NK cells to secrete a variety of cytokines. However, further studies have found that IL-2 can cause overdifferentiation of T cells and induce apoptosis of activated T cells, and can also activate CD4+FoxP3Treg regulatory cells, thereby inhibiting activation of T cells and tumor killing activity. Therefore, IL-2 is considered to be a T cell regulatory factor rather than just an activation factor, so some studies have now used IL-7, IL-15, and IL-21 to replace IL-2.

Specifically, interleukin 7 (IL-7): IL-7 is a hematopoietic growth factor, secreted by stromal cells in the bone marrow and thymus, and shares the $\gamma c$ receptor subunit with IL-2 to stimulate the proliferation of lymphocyte progenitor cells. IL-7 can provide continuous stimulation signals for Naive T cells and memory T cells. As mentioned above, IL-7 will not activate CD4+ FoxP3+ Treg cells during the activation of CD8+ T cells. In clinical practice, IL-7 can also be used to restore the number of T cells after chemotherapy or hematopoietic stem cell transplantation. And IL-7 plays an important role in certain stages of maturation and can affect the proliferation of B cells. IL-7 can also act as a regulatory factor for intestinal mucosal lymphocytes.

Specifically, interleukin 15 (IL-15): IL-15 has a similar structure to IL-2, shares the $\gamma c$ receptor subunit, and belongs to the family with 4 $\alpha$-helix bundles (others include, such as, IL-2, IL-4, IL-7, IL-9, G-CSF and GM-CSF). IL-15 regulates the activation and proliferation of T cells and NK cells. IL-15 mainly kills virus-infected cells in the innate immune system. Also, IL-15 can activate NKT cells and $\gamma\delta T$ cells. In immune cell therapy, IL-15 does not cause apoptosis of activated T cells, but activates CD8+ effector T cells. IL-15 maintains the survival of memory T cells, thus playing an important role in long-term anti-tumor activity.

Specifically, interleukin 21 (IL-21): IL-21 also belongs to the IL-2 family, shares the $\gamma c$ receptor subunit, has a strong regulatory effect on the cells of the immune system, and can induce cell division and proliferation in its target cells. In cellular immunotherapy, IL-21 can promote the proliferation of CD4+ and CD8+T cells, enhance the cytotoxicity of CD8+T cells and NK cells, and will not cause cell apoptosis due to activation. IL-21 will preferentially expand "young" CD8+ T cells with CD27+ and CD28+, which have stronger cytotoxicity. Of course, IL-21 will not cause the expansion of Treg, so IL-21 is increasingly used in cell immunotherapy.

Specifically, interleukin 4 (IL-4): IL-4 activates the proliferation of activated B cells and T cells, and regulates the expression of Fc receptors on lymphocytes and monocytes. IL-4 induces the transformation of Th1 cells to Th2 cells. IL-4 stimulates Th2 cells to secrete IL-4, IL-5, IL-6, IL-10 and IL-13. IL-4 guides monocytes to differentiate into DCs by inhibiting the growth of macrophages. When IL-4 is not added to a culture system, monocytes will differentiate into macrophages. IL-4 plays a key role in regulating humoral immunity and adaptive immunity, inducing B cell antibody class switching to IgE and upregulating the production of MHC class II molecules. The combined action of IL-4 and GM-CSF can direct the differentiation of monocytes into immature DCs. At this time, DCs have stronger antigen uptake and processing capabilities, but weaker antigen presentation capabilities. The sequential use of IL-4 and TNF-$\alpha$ can promote DC maturation.

Specifically, interleukin 12 (IL-12): IL-12 acts on activated T and NK cells, has a wide range of biological activities, and acts on lymphocytes through the mediation of the activator of the transcription protein STAT4. IL-12 is required for the T cell-independent induction of IFN-$\gamma$ and plays an important role in the differentiation of Th1 and Th2 cells. IL12B combines with IL23A to form IL-23 interleukin, which has innate and adaptive immune functions. IL-12 is a drug target. In cellular immunotherapy, IL-12 promotes the differentiation of CD4+ T cells into CD4+ Th1 T cells and enhances the activity of CD8+ CTLs cells. The curative effect of IL-12 is related to its dose, duration of action, and other interacting cytokines, and promotes the tumor-killing activity of immune cells through multiple mechanisms. In the mouse anti-melanoma model, high-dose IL-12 acts through NK cells, while low-dose IL-12 has a tumor-killing effect through NKT.

Specifically, interleukin 18 (IL-18): IL-18 is also called interferon-$\gamma$ inducing factor, which is a proinflammatory cytokine produced by macrophages and other cells. IL-18 can stimulate NK cells and CD8+ T cells to secrete IFN-$\gamma$, and enhance the cytotoxic effect of NK cells and CD8+ T cells. IL-18 can also activate macrophages, promote the development of Th1 CD4+ T cells and promote the expression of FasL by lymphocytes. IL-18 can provide a potential therapeutic target for allergic diseases. In addition, the synergistic effect of IL-18, IL-12 and IL-15 can maintain Th1 response and monokine production in autoimmune diseases.

Interferon-$\gamma$ (IFN-$\gamma$) belongs to type II interferon, which is mainly produced by NK and NKT cells, has antiviral, antitumor and immunomodulatory effects and includes IFN-$\gamma$ and IFN-$\beta$. IFN-$\gamma$ has an anti-proliferative effect on transformed cells and can enhance the antiviral and anti-tumor effects of type I interferon. By activating macrophages, IFN-$\gamma$ induces the expression of MHC I, MHCIII and co-activator molecules on antigen presenting cells (APCs). In addition, IFN-$\gamma$ can induce changes in the expression of proteasomes to enhance antigen presentation ability. IFN-$\gamma$ can also promote the differentiation of CD4+ T cells into Th1 cells and inhibit the subtype switching of B cells that depends on IL-4. IFN-$\gamma$ activates the JAK-STAT cell pathway by phosphorylating JAK1 and JAK2 proteins. In cellular immunotherapy, IFN-$\gamma$ acts on host immune cells and has certain effects on macrophages, T cells, B cells and NK cells. IFN-$\gamma$ enhances antigen

presentation ability by promoting the expression of MHC class II molecules in macrophages or by causing some cells that normally do not express MHC class II molecules (such as vascular endothelial cells, some epithelial cells and connective tissue cells) to express MHC class II molecules. IFN-$\gamma$ can promote the differentiation of B cells and CD8+T cells, but cannot promote their proliferation. IFN-$\gamma$ can enhance the activity and immune function of TH1 cells. IFN-$\gamma$ enhances the phagocytic ability of neutrophils and activates NK cells to enhance their cytotoxic effect. Abnormal expression of IFN-$\gamma$ is associated with many autoinflammatory and autoimmune diseases.

Tumor necrosis factor belongs to the TNF superfamily cytokines and is a multifunctional molecule that regulates biological processes, including cell proliferation, differentiation, apoptosis, lipid metabolism and coagulation. TNF-$\alpha$ participates in anti-tumor. In terms of cellular immunotherapy, TNF-$\alpha$ causes immature DC to differentiate into mature DC. This process is achieved by TNF-$\alpha$ downregulating the macropinocytosis of immature DC and the expression of surface Fc receptors, and upregulating the expression of MHC class I and class II molecules and B7 family molecules (CD80, CD86, etc.) on cell surface. The antigen uptake and processing capabilities of mature DCs are significantly weakened, but their antigen presentation capabilities are significantly enhanced, which can strongly activate T cells. TNF-$\alpha$ can also affect the production of other cytokines, such as stimulating the secretion of IL-1 by monocytes and macrophages, enhancing the proliferation of IL-2-dependent thymocytes and T cells, promoting the production of lymphokines such as IL-2, CSF and IFN-$\gamma$, and enhancing the proliferation and Ig secretion of B cells stimulated by mitogens or foreign antigens.

Granulocyte macrophage colony-stimulating factor (GM-CSF) plays a key role in embryo transfer and development. GM-CSF is one of the earliest cytokines discovered to have an effect on DCs. In DC culture, GM-CSF promotes the differentiation of monocytes into large macrophage-like cells and promotes the expression of MHC class II molecules on the cell surface, thereby enhancing the antigen presentation function of cells. In addition, GM-CSF can also promote DC survival. In terms of cellular immunotherapy, GM-CSF can activate immune responses and produce anti-tumor activity by activating macrophages and DCs. In terms of antigen presentation, GM-CSF can promote DC cell maturation, promote the upregulation of costimulatory molecules and the expression of CD1d receptors. Recent studies have found that GM-CSF stimulates the differentiation of hematopoietic progenitor cells into monocytes and neutrophils, thereby reducing the risk of febrile neutropenia in cancer patients. Other studies have shown that GM-CSF can induce the differentiation of bone marrow DCs, promote Th1 cell-biased immune responses and angiogenesis, and affect the development of allergic inflammation and autoimmune diseases. Therefore, GM-CSF is used clinically to treat malignant tumors.

The term "nucleic acid molecule", as used herein, generally refers to nucleotides of any length. In the present application, the term "nucleic acid molecule" may encode a protein included by the oncolytic virus. In the present application, the nucleic acid molecule may include DNA and/or RNA. In some cases, the RNA may include single-stranded RNA (ssRNA) or double-stranded RNA (dsRNA). The single-stranded RNA may include sense RNA or anti-sense RNA, or it may include ambisense RNA.

The term "prevention", as used herein, generally refers to preventing the occurrence and onset, recurrence, and/or spread of a disease or one or more symptoms of the disease by taking certain measures in advance. The term "treatment", as used herein, generally refers to eliminating or ameliorating a disease, or one or more symptoms associated with the disease. In certain embodiments, treatment generally refers to administering one or more drugs to a patient suffering from the disease so that the disease is eliminated or alleviated. In certain embodiments, "treatment" may be the administration of the pharmaceutical composition and/or drug product in the presence or absence of other drugs after the onset of symptoms of a particular disease. For example, it may be the use of the pharmaceutical composition and/or drug product described herein to prevent the occurrence, development, recurrence and/or metastasis of a tumor.

The term "tumor", as used herein, generally refers to any new pathological growth of tissue. The tumor may be benign or malignant. In the present application, the tumor may be a solid tumor or a hematological tumor. For research use, these tissues can be isolated from readily available sources by methods well known to those skilled in the art.

In some specific embodiments, the tumors include but are not limited to acute lymphoblastic leukemia, acute B-cell leukemia, chronic non-lymphocytic leukemia, non-Hodgkin's lymphoma, anal cancer, astrocytoma, basal cell carcinoma, bile duct cancer, bladder cancer, mastocarcinoma, breast cancer (BRCA), cervical cancer, chronic myeloproliferative tumors, colorectal cancer, endometrial cancer, ependymoma, esophageal cancer, diffuse large B-cell lymphoma (DLBCL), sensorineuroblastoma, Ewing's sarcoma, fallopian tube cancer, gallbladder cancer, gastric cancer, gastrointestinal carcinoid tumors, hepatocellular carcinoma, hypopharyngeal cancer, Kaposi sarcoma, kidney cancer, Langerhans cell hyperplasia, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumors, pharyngeal cancer, pituitary tumors, prostate cancer, rectal cancer, renal cell carcinoma, retinoblastoma, skin cancer, small cell lung cancer, small intestine cancer, squamous neck cancer, testicular cancer, thymoma, thyroid cancer, uterine cancer, vaginal cancer and vascular tumors.

## DETAILED DESCRIPTION

A wild-type VSV, in particular, an Indiana strain of the VSV, Indiana MuddSummer subtype strain of the VSV, is provided. The Indiana MuddSummer subtype strain of the VSV includes: an M protein with an amino acid sequence as shown in SEQ ID NO 1; a G protein with an amino acid sequence as shown in SEQ ID NO 12; an N protein with an amino acid sequence as shown in SEQ ID NO 14; a P protein with an amino acid sequence as shown in SEQ ID NO 16; an L protein with an amino acid sequence as shown in SEQ ID NO 18. In the present application, the M protein, G protein, N protein, P protein, and L protein all can be modified.

A recombinant oncolytic virus is provided. The oncolytic virus is derived from the wild-type VSV described above and obtained by mutating the sites on the amino acid sequences of the M protein, G protein, N protein, P protein, and L protein of the wild-type VSV.

The present application provides a method for treating tumors using a combination of an oncolytic virus vaccine and immune cells, which specifically includes:
treating the tumors using the combination of the immune cells and the oncolytic virus vaccine.

The oncolytic virus vaccine includes a recombinant oncolytic virus expressing a tumor antigen and is used for targeting tumor cells (MARK). The recombinant oncolytic virus can not only kill tumor cells on its own, but also can target tumor cells, transforming "tumors that have no target but need to be treated" into "tumors that have targets and can be treated".

The immune cells express a chimeric antigen receptor paired with the tumor antigen, and are used for killing or destroying the tumor cells targeted, achieving a curative effect where 1 +1 is greater than 2.

The recombinant oncolytic virus includes an M protein, a G protein, an N protein, a P protein, and an L protein.

Compared to an amino acid sequence shown in SEQ ID NO 1, the M protein includes site mutations of M51R, V221F, and S226R; or the M protein includes site mutations of N32S, N49D, M51R, H54Y, V221F, V225I, and S226R; or the M protein includes site mutations of N32S, N49D, M51R, H54Y, knockouting of bases encoding leucine at position 111, V221F, V225I, and S226R; or the M protein includes site mutations of N32S, N49D, M51R, H54Y, L111A, V221F, V225I, and S226R; or the M protein includes site mutations of G21E, N32S, N49D, M51R, H54Y, V221F, V225I, and S226R; or the M protein includes site mutations of G21E, N32S, M33A, N49D, M51R, H54Y, V221F, V225I, and S226R; or the M protein includes site mutations of G21E, N32S, M33A, N49D, M51R, H54Y, A133T, V221F, V225I, and S226R; or the M protein includes site mutations of N32S, M33A, N49D, M51R, H54Y, V221F, V225I, and S226R; or the M protein includes site mutations of N32S, M33A, N49D, M51R, H54Y, A133T, V221F, V225I, and S226R; or the M protein includes site mutations of N32S, N49D, M51R, H54Y, A133T, V221F, V225I, and S226R.

Compared to an amino acid sequence shown in SEQ ID NO 12, the G protein includes site mutations of V53I, A141V, D172Y, K217E, D232G, V331A, V371E, G436D, T438S, F453L, T471I, and Y487H.

Compared to an amino acid sequence shown in SEQ ID NO 14, the N protein includes site mutations of I14V, R155K, and S353N.

Compared to an amino acid sequence shown in SEQ ID NO 16, the P protein includes site mutations of R50K, V76A, D99E, L126S, L140S, H151Y, I168M, K170E, Y189S, and N237D.

Compared to an amino acid sequence shown in SEQ ID NO 18, the L protein includes site mutations of S87P and I487T.

Further, the recombinant oncolytic virus is obtained by introducing an exogenous gene encoding the tumor antigen into the recombinant oncolytic virus described above.

Further, the tumor antigen is selected from a hematological tumor antigen and a solid tumor antigen.

Further, the tumor antigen is one or more selected from a group consisting of: CD19, CD22, BCMA, MUC1, NY-ESO-1, MAGE A4, cMet, Claude 18.2, MSLN, EGFR, VEGFR2, and HER-2.

Further, the immune cells are selected from T cells, NK cells and M cells.

Further, the immune cells are selected from CAR-T cells, TCR-T cells, CAR-γδ-T cells, CAR-Crispered-T cells, STAR-T cells, CAR-NK cells, and CAR-M cells.

Further, the recombinant oncolytic virus further includes a cytokine encoded by an exogenous gene.

Further, the cytokine is selected from interleukins (IL), interferons (IFN), tumor necrosis factors (TNF), colony stimulating factors (CSF), transforming growth factor-β family (TGF-β family), and chemokine family, growth factors.

Further, the cytokine is one or more selected from a group consisting of: GM-CSF, IL-2, IL-12, IL-15, IL-18, TNF-α, and IFN-β.

The recombinant oncolytic virus includes a nucleic acid molecule; the nucleic acid molecule includes a nucleic acid sequence encoding the M protein with the site mutations, a nucleic acid sequence encoding the G protein with the site mutations, a nucleic acid sequence encoding the N protein with the site mutations, a nucleic acid sequence encoding the P protein with the site mutations, a nucleic acid sequence encoding the L protein with the site mutations, a nucleic acid sequence encoding the tumor antigen, and a nucleic acid sequence encoding the cytokine.

In the present application, the recombinant oncolytic virus described herein can be obtained by a virus packaging process and a virus rescue process. The specific process may include infecting and inoculating BSR-T7 cells with poxvirus vTF7-3 expressing T7 RNA polymerase, and performing lipofectamine transfection using expression plasmids that clone VSV N, VSV P, and VSV L genes, respectively and backbone plasmids, to obtain the oncolytic virus of interest.

The present application provides a composition; the composition includes the recombinant oncolytic virus and the immune cells described above.

In certain embodiments, the composition may include a suitable formulation of one or more (pharmaceutically effective) adjuvants, stabilizers, excipients, diluents, solubilizers, surfactants, emulsifiers and/or preservatives. The acceptable components of the composition are preferably non-toxic to a recipient at the dose and concentration used. The compositions of the present application include but are not limited to liquid, frozen and lyophilized compositions.

In certain embodiments, the pharmaceutically acceptable carrier may include any and all solvents, dispersion media, coatings, isotonic agents and absorption delaying agents that are compatible with drug administration, and are generally safe and non-toxic.

In certain embodiments, the composition may be useful for administration including parenteral, subcutaneous, intracavitary, intraarterial, intravenous, intrathecal, and/or intranasal administration or may be directly injected into tissues. For example, the composition may be administered to a patient or subject by infusion or injection. In certain embodiments, the administration of the composition may be performed in different ways, such as intravenous, intraperitoneal, subcutaneous, intramuscular, intradermal or intratissue administration. In certain embodiments, the composition may be administered uninterruptedly. The uninterrupted (or continuous) administration may be achieved by a small pump system worn by a patient to measure a therapeutic agent flowing into the patient's body, as described in WO2015/036583.

The present application further provides a use of the composition described above in preparing a medicine for preventing and/or treating a disease and/or disorder.

In the method for treating tumors using a combination of a recombinant oncolytic virus vaccine and immune cells provided by the present application, the recombinant oncolytic virus in the oncolytic virus vaccine is obtained by respectively performing site-directed mutagenesis on the amino acids of the M protein, G protein, N protein, P protein, and L protein of an oncolytic virus, and further inserting a tumor antigen and/or a cytokine encoded by an exogenous gene. Meanwhile, the immune cells express the chimeric antigen receptor for killing or destroying tumor cells, thereby further improving the infection ability of the oncolytic virus to abnormally proliferative (tumor) LLC cells. Moreover, the recombinant oncolytic viruses prepared have a poor infection ability to normal cells (normal MEF cells), indicating that the recombinant oncolytic viruses prepared herein can be well used for infecting tumors, cancers and other cells without damaging normal cells, and have broad application prospects.

The recombinant oncolytic virus in the oncolytic virus vaccine of the present application is not easily eliminated in the abnormally proliferative (tumor) LLC cells. Relatively speaking, wild-type oncolytic viruses are more easily eliminated in the LLC cells. Since the recombinant oncolytic virus of the present application is obtained by performing site-directed mutagenesis on the amino acids of the M protein, G protein, N protein, P protein, and L protein of an oncolytic virus, respectively and further inserting an antigen and/or cytokine encoded by an exogenous gene, the oncolytic virus is more difficultly eliminated in the LLC cells, thereby further ensuring that the oncolytic virus can better exert the infection ability and killing ability to the LLC cells. Moreover, the recombinant oncolytic virus of the present application is more easily eliminated in normal MEF cells and the safety of the normal MEF cells is further ensured, thereby improving the safety of the recombinant oncolytic viruses.

The present application is further described in detail below in combination with Preparation Examples 1-113 and Examples.

## PREPARATION EXAMPLES

Preparation Examples 1-8

Preparation Example 1

Preparation Example 1 provides a method for constructing CAR-T cells. The method specifically included the following steps:

(1) Production of retrovirus for CAR expression

A CD19-CAR sequence was constructed and then inserted into a lentivirus packaging system. After transfection of each plasmid into Phoenix ECO cell line (ATCC) using Lipofectamin 3000 (Invitrogen), a culture supernatant containing ecotropic retrovirus secreted for 24-48 h was added to PG13 retroviral packaging cell line (ATCC) and spin infection (2500 rpm, 90 min) was performed.

A culture supernatant of the PG13 retrovirus-producing cell line prepared by the above method was harvested and filtered (0.45 $\mu$m filter) to remove residual cell particles, and then concentrated 4 times using a centrifugal filtration device (Millipore Amicon 100KD cut-off) to obtain CD19-CAR retrovirus, which was used as a retrovirus concentrate for construction of CAR-T cells.

(2) Preparation of CAR-T cells

Leukocytes obtained by leukapheresis of normal human leukocyte components were added to a 24-well plate coated with anti-CD3 antibody (OKT3, 10 μg/ml, BioXcell) together with anti-CD28 antibody (CD28.2, 2 μg/ml, BD Biosciences), and cultured for 48 h to activate T cells. Activated T cells were washed twice and used for retrovirus transduction.

After overnight coating of a 24-well plate with Retronectin (20 μg/ml, TaKaRa) at 4°C, 2% BSA-DPBS was added to the 24-well plate washed, followed by blocking at 37°C for 30 min. After washing, 1 ml of the retrovirus concentrate was added, followed by centrifugation at 2000×g, 32°C for 2 h to adhere the retroviruses to the bottom of the wells. After removal of the retrovirus concentrate and washing of the wells, 1 ml of the activated T cells ($1 \times 10^6$ cells/ml) were added to the wells, followed by centrifugation at 1000×g, 32°C for 10 min to adhere the cells to the retroviruses.

Subsequently, the cells were incubated for 48 h in the presence of human IL-2 (300 IU/ml, Proleukin, Novartis). In this way, retrovirus-transduced T cells were obtained, then the retrovirus-transduced T cells were washed twice, and proliferated with a fresh culture medium containing human IL-2 (200 IU/ml) for 3 to 6 days to be used as CAR-T cells.

With regard to the CAR protein expression on the cell surface, CAR-T cells proliferated for 3 days after retrovirus transduction were stained with CD19-Ck protein (fusion protein of CD19 extracellular domain and human immunoglobulin κ chain constant region (Ck)) and anti-Ck-APC (BioLegend), and then detected by flow cytometry (FACS-Calibur, BD Biosciences).

According to the detection results, CD19-CAR-T cells were successfully constructed. The amino acid sequence of the CD19 CAR-T cells (3rd generation) is shown in SEQ ID NO 66.

According to the construction method described in Preparation Example 1, other tumor antigen-CAR-T cells may be obtained by replacing CD19 with other types of tumor antigen. The type of tumor antigen may also be CD22, BCMA, MUC-1, cMet, Claude18.2, MSLN, EGFR, VEGFR2, HER-2, TPBG, AFP, etc. A person skilled in the art may construct other CAR-T cells by the same method except for different CARs loaded on the T cells, and detect the success of construction by the same method.

When the tumor antigen was CD22, BCMA, MUC-1, cMet, Claude18.2, MSLN, EGFR, VEGFR2, HER-2 or TPBG respectively, CD22-CAR-T cells, BCMA-CAR-T cells, MUC-1-CAR-T cells, cMet-CAR-T cells, Claude18.2-CAR-T cells, MSLN-CAR-T cells, EGFR-CAR-T cells, VEGFR2-CAR-T cells, HER-2-CAR-T cells, TPBG-CAR-T cells, and AFP-CAR-T cells were obtained, respectively.

In a specific embodiment, the amino acid sequence of antigen receptor CD19 paired with the tumor antigen is shown in SEQ ID NO 37.

In a specific embodiment, the amino acid sequences of the heavy chain variable region and light chain variable region of antigen receptor CD22 paired with the tumor antigen are shown in SEQ ID NO 38 and SEQ ID NO 39, respectively.

In a specific embodiment, the amino acid sequences of the heavy chain variable region and light chain variable region of antigen receptor BCMA paired with the tumor antigen are shown in SEQ ID NO 40 and SEQ ID NO 41, respectively.

In a specific embodiment, the amino acid sequences of the heavy chain variable region and light chain variable region of antigen receptor cMet paired with the tumor antigen are shown in SEQ ID NO 42 and SEQ ID NO 43, respectively.

In a specific embodiment, the amino acid sequences of the heavy chain variable region and light chain variable region of antigen receptor Claude 18.2 paired with the tumor antigen are shown in SEQ ID NO 44 and SEQ ID NO 45, respectively.

In a specific embodiment, the amino acid sequences of the heavy chain variable region and light chain variable region of antigen receptor MSLN paired with the tumor antigen are shown in SEQ ID NO 46 and SEQ ID NO 47, respectively.

In a specific embodiment, the amino acid sequences of the heavy chain variable region and light chain variable region of antigen receptor EGFR paired with the tumor antigen are shown in SEQ ID NO 48 and SEQ ID NO 49, respectively.

In a specific embodiment, the amino acid sequences of the heavy chain variable region and light chain variable region of antigen receptor VEGFR2 paired with the tumor antigen are shown in SEQ ID NO 50 and SEQ ID NO 51, respectively.

In a specific embodiment, the amino acid sequences of the heavy chain variable region and light chain variable region of antigen receptor HER-2 paired with the tumor antigen are shown in SEQ ID NO 52 and SEQ ID NO 53, respectively.

In a specific embodiment, the amino acid sequences of the heavy chain variable region and light chain variable region of antigen receptor TPBG paired with the tumor antigen are shown in SEQ ID NO 54 and SEQ ID NO 55, respectively.

In a specific embodiment, the amino acid sequences of the heavy chain variable region and light chain variable region of antigen receptor AFP paired with the tumor antigen are shown in SEQ ID NO 56 and SEQ ID NO 57, respectively.

In a specific embodiment, the amino acid sequences of antigen receptor PD-L1 paired with the tumor antigen is shown in SEQ ID NO 72, and the amino acid sequences of PD-L1 CAR-T cells (3rd generation) is shown in SEQ ID NO 73.

Preparation Example 2

Preparation Example 2 provides a method for constructing TCR-T cells. The method specifically included the following steps:

(1) Cloning and sequencing of specific T cells

A chemically synthesized tumor-specific oligopeptide or specific antigen (NY-ESO-1) was used to stimulate peripheral blood mononuclear cells (PBMC) derived from specific genotypes in vitro. After 2 rounds of polypeptide stimulation, polyclonal T cells were stimulated with the tumor-specific oligopeptide or specific antigen, and co-cultured overnight at 37°C, and target T cells positive for T cell activation markers were sorted out by flow cytometry.

After centrifugation for removing supernatant, T cells ($1 \times 10^6$) were resuspended in 1 mL Trizol (RNeasy Plusu Universal Mini Kit, QIAGEN), quickly frozen in liquid nitrogen and sent to CRO company for sequencing (immunobank sequencing).

According to the sequencing results, TCRα chain and TCRβ chain were paired, and full-length TCR containing constant regions was constructed by PCR (elements of the TCR sequence: TCRα variable region-TCRα, constant region-P2A-TCRβ, variable region-TCRβ constant region) and inserted into a retrovirus vector.

(2) Preparation of specific TCR-T cells

Target T cells were inserted into retrovirus vector pMSGV1 (addgene) to construct a pMSGV1-TCR vector. Retroviral packaging cell line 293GP cells were transfected with pMSGV1-TCR and pVSV-G plasmids to prepare a retrovirus and T cells were transduced with virus supernatant.

Transfection was performed as follows: On day 0, 293GP cells were seeded into 6-well plates ($6 \times 10^5$ cells/well). On day 1, 293GP cells were transfected with pMSGV1-TCR and pVSV-G plasmids (2μg pMSGV1-TCR and 1.4μg pVSV-G/well) and on the same day, PBMC from healthy individuals were activated with anti-human CD3 antibody (OKT3). On day 3, culture medium containing virus supernatant was collected and fresh culture medium (DMEM supplemented with 10% fetal bovine serum) was added to 293GP cells, and the activated T cells were transfected with virus supernatant collected by centrifugation. On day 4, the activated T cells were transfected in the same way for the second time. On day 5, the transfected T cells were collected and cultured in T25 culture flasks (culture medium was X-VIVO (Lonza) supplemented with 10% fetal bovine serum and 300 IU/mL IL2). On day 10, the expression level of target TCR was detected by flow cytometry, and the positive rate of TCR-T cells was detected by detecting the expression rate of TCRβ chain constant region (TRBC) by antibody.

According to the detection results, NY-ESO-1 TCR-T cells were successfully constructed.

According to the construction method described in Preparation Example 2, other tumor antigen-CAR-T cells may be obtained by replacing NY-ESO-1 with other types of the tumor antigen. The types of tumor antigen may also be MAGE A4, AFP, MAGE-A10, MAGE-1, MAGE-B2, MAGE-3, MAGE-6, BAGE, GAGE-1, GAGE-2, GAGE-8, GAGE-3, GAGE-4, GAGE-5, GAGE-6, GAGE-7b, SAGE, HAGE, SSX, SCP1, LAGE, and a melanoma differentiation antigen, including Melan-A, Mart-1, gp100, gp75, TRP21, and TRP22. A person skilled in the art may construct other TCR-T cells by the same method except for different TCRs loaded on the T cells, and detect the success of construction by the same method.

When the tumor antigen was MAGE A4, AFP, MAGE-A10, MAGE-1, MAGE-2, MAGE-3, MAGE-6, BAGE, GAGE-1, GAGE-2, GAGE-8, GAGE-3, GAGE-4, GAGE-5, GAGE-6, GAGE-7b, SAGE, HAGE, SSX, SCP1, LAGE, or a melanoma differentiation antigen, including Melan-A, Mart-1, gp100, gp75, TRP21, and TRP22 respectively, MAGE A4 TCR-T cells, AFP-1 TCR-T cells, MAGE-A10 TCR-T cells, MAGE-1 TCR-T cells, MAGE-2 TCR-T cells, MAGE-3 TCR-T cells, MAGE-6 TCR-T cells, BAGE TCR-T cells, GAGE-1 TCR-T cells, GAGE-2 TCR-T cells, GAGE-8 TCR-T cells, GAGE-3 TCR-T cells, GAGE-4 TCR-T cells, GAGE-5 TCR-T cells, GAGE-6 TCR-T cells, GAGE-7b TCR-T cells, SAGE TCR-T cells, HAGE TCR-T cells, SSX TCR-T cells, SCP1 TCR-T cells, LAGE TCR-T cells, Melan-A TCR-T cells, Mart-1 TCR-T cells, gp100 TCR-T cells, gp75 TCR-T cells, TRP21 TCR-T cells, and TRP22 TCR-T cells were obtained, respectively.

In a specific embodiment, the amino acid sequences of the TCRα chain and TCRβ chain of the antigen receptor NY-ESO-1 paired with the tumor antigen are shown in SEQ ID NO 58 and SEQ ID NO 59, respectively.

In a specific embodiment, the amino acid sequences of the TCRα chain and TCRβ chain of the antigen receptor MAGE A4 paired with the tumor antigen are shown in SEQ ID NO 60 and SEQ ID NO 61, respectively.

In a specific embodiment, the amino acid sequences of the TCRα chain and TCRβ chain of the antigen receptor AFP paired with the tumor antigen are shown in SEQ ID NO 62 and SEQ ID NO 63, respectively.

In a specific embodiment, the amino acid sequences of the TCRα chain and TCRβ chain of the antigen receptor MAGE B2 paired with the tumor antigen are shown in SEQ ID NO 64 and SEQ ID NO 65, respectively.

Preparation Example 3

Preparation Example 3 provides a method for constructing CAR-γδ-T cells. The method specifically included the following steps:

(1) Lentivirus packaging

Plasmid transfection: CD19-CAR sequence was constructed and inserted into lentivirus packaging system; plasmid, PEI and culture medium of Opti-MEM were placed at room temperature for 5 min, respectively; 436 µl of Opti-MEM was placed into a 1.5 ml EP tube, 64 µl of PEI was then added, a first resulting solution was well mixed and allowed to stand at room temperature for 5 min to obtain a PEI-Opti-MEM solution; pLP1, pLP2, pLP-BaEVTR, and the expression plasmid were added into a tube at a ratio of 6:9:9:16, and Opti-MEM was then added to 500 µl, and a second resulting solution was allowed to stand at room temperature for 5 min; the PEI-Opti-MEM solution prepared was added into the Opti-MEM containing the plasmid and a third resulting solution was allowed to stand at room temperature for 20 min to obtain a DNA/PEI mixture; 1 ml of DNA/PEI mixture was slowly dropwise added into 293T cells, gently mixed, incubated in an incubator at 37°C for 16-18 h, replaced with fresh medium, and further incubated in a 5% $CO_2$ incubator at 37°C to obtain CD19-CAR retrovirus.

Virus collection and concentration: 48h after plasmid transfection, supernatant was collected, centrifuged at 4°C, 2820 g for 20 min, cell debris was then removed, and the supernatant was filtered with a 0.45 µm filter; a filtered virus supernatant was centrifuged at 50000 g for 2 h in an ultrafiltration centrifugal tube, a resulting supernatant was removed, and an appropriate amount of medium was added to dissolve pellets, thus obtaining a virus solution; the virus solution was subpackaged into cryopreservation tubes and stored at -80°C; and 200 µl of virus solution was taken for titer determination.

Virus titer detection: $1 \times 10^5$ 293T cells were seeded in a 24-well plate, different volumes of the virus solution were added, the cells were incubated in a 5% $CO_2$ incubator at 37°C for 72 h, and then the positive rate of cells was detected to calculate the virus titer.

(2) Preparation of CAR-γδ-T cells

Isolation of PBMC: 50 ml of peripheral blood was collected. Under normal working condition of an ultra-clean bench, lymphocyte separation medium was added into two 50 ml sterilized centrifuge tubes, 15 ml each. The peripheral blood was slowly injected into the upper layer of lymphocyte separation medium in the two centrifuge tubes, 25-30 ml each. Centrifugation (700g × 20 min, speed up 1, speed down 2, room temperature) was performed. After centrifugation, the blood was divided into four layers, consisting of plasma (upper layer), mononuclear cells between the plasma and the separation medium (second layer), the separation medium (third layer), and a red blood cell layer (bottom layer). The mononuclear cells at the second layer were collected into a new centrifuge tube with a pipette. The cell suspension was diluted with 20 ml of PBS and centrifuged at 500g for 10 min.

Stimulation of γδ-T cells on Day 0: Supernatant was removed, PBMC was resuspended in 10 ml of GT-T551 H3 medium to obtain a cell suspension, and 20 µl of the cell suspension was taken for counting. The above cell suspension was seeded into a cell culture flask at $2 \times 10^6$ cells/mL, and 10% (v/v) FBS, zoledronic acid at a final concentration of 5 µM, IL2 at a final concentration of 500 IU/mL, and IL21 at a final concentration of 10 ng/mL were added.

Virus infection: On Day3, a non-tissue culture 24-well plate was coated with RetroNectin. Specifically, Retro-Nectin stock solution was diluted to 40 ug/mL with PBS, and 1 mL of the diluted RetroNectin was added to the 24-well plate; the plate was blocked with a sealing film for incubation overnight at 4°C. On Day 4, RetroNectin was removed, the plate was blocked with 2% human serum albumin (0.5 mL/well) for 30 min at room temperature, and then washed once with PBS; lentivirus (the virus solution obtained in setp (1)) was added to the coated wells, and the culture plate was centrifuged at 1500 g, 32°C for 2 h; the medium used was GT-T551H3 medium, supplemented with 10% (v/v) FBS, IL2 at a final concentration of 500 IU/mL, and IL21 at a final concentration of 10 ng/mL (unless otherwise specified, the medium was used later). The lentivirus was then removed and the wells were washed once with PBS (the PBS was removed when the cells were added). The γδT cells collected by centrifugation were resuspended to $1 \times 10^6$ cells/mL (500 uL/well, i.e., 5E5 cells/well) in fresh complete culture medium, and then added into the wells; the cells were shaken thoroughly, and the well plates were centrifuged at 32°C, 1500 g for 30 min to 2 h, followed by incubation in an incubator at 37°C.

On Day 5, the above operations: coating, loading and infection, were repeated again.

On Day 6, cells from each well were collected, centrifuged, washed once with PBS, resuspended in complete medium, and seeded into new well plates at a density of $1\text{-}1.5\times10^6$ cells/mL.

$\alpha\beta$T cell depletion: The cells were collected, counted, washed with pre-cooled DPBS, centrifuged and resuspended in working buffer (PBS, pH 7.2, 0.5% BSA, 2 mM EDTA), added with 10 $\mu$L of anti-TCR$\gamma$/$\delta$Hapten-Antibody/107 cells, incubated at 4°C for 10 min, added with working buffer and MACS anti-Hapten MicroBeads-FITC, well mixed and incubated at 4 °C for 15 min, washed with working buffer, centrifuged and then resuspended in 500 $\mu$L working buffer, thus obtaining CD19-CAR-$\gamma\delta$-T cells.

Detection of CAR expression rate: CAR-$\gamma\delta$-T cells and $\gamma\delta$-T cells without virus transduction (Mock control cells) were taken on D10, D12, D14, D16 and D18, and stained with Protein L. The results showed that the positive rate of CAR-$\gamma\delta$-T cells was between 35% and 50%, and the expression was stable during culture, and slightly decreased on D18; Mock control cells did not express CAR.

According to the detection results, CD19-CAR-$\gamma\delta$-T cells were successfully constructed.

According to the construction method described in Preparation Example 3, other tumor antigen- CAR-$\gamma\delta$-T cells may be obtained by replacing CD19 with other types of tumor antigen. The types of tumor antigen may also be CD22, BCMA, MUC-1, cMet, Claude18.2, MSLN, EGFR, VEGFR2, HER-2, TPBG, etc. A person skilled in the art may construct other CAR-$\gamma\delta$-T cells by the same method except for different CARs loaded on the T cells, and detect the success of construction by the same method.

When the tumor antigen was CD22, BCMA, MUC-1, cMet, Claude18.2, MSLN, EGFR, VEGFR2, HER-2 or TPBG respectively, CD22-CAR-$\gamma\delta$-T cells, BCMA -CAR-$\gamma\delta$-T cells, MUC-1-CAR-$\gamma\delta$-T cells, cMet-CAR-$\gamma\delta$-T cells, Claude 18.2-CAR-$\gamma\delta$-T cells, MSLN-CAR-$\gamma\delta$-T cells, EGFR-CAR-$\gamma\delta$-T cells, VEGFR2-CAR-$\gamma\delta$-T cells, HER-2-CAR-$\gamma\delta$-T cells, and TPBG-CAR-$\gamma\delta$-T cells were obtained, respectively.

Preparation Example 4

Preparation Example 4 provides a method for constructing CAR-Crispered-T cells. The method specifically included the following steps:

(1) Immune-checkpoint gene knockout of T cells

According to the sequence structures of human PD-1, CTLA4, LAG-3 and Tim-3 genes, and referring to the existing research contents of relevant CRISPR gene editing tools, respective sgRNA sequences were designed. The sgRNA of PD-1 gene was gaggaccgcagcc, the sgRNA of CTLA4 gene was ctgcaagcaatgcacg, the sgRNA of LAG-3 gene was ggggccagg, and the sgRNA of Tim-3 gene was ggtcatcaaccagcca. The nucleic acid sequences of sgRNA were synthesized by Sangon Biotech (Shanghai) Co., Ltd. and inserted into standard vectors respectively and ligated into CRISPR/CAS9 expression vector pX330A to obtain pX330A-PD-1, pX330A-CTLA4, pX330A-LAG-3 and pX330A-Tim-3 vectors.

T cells were electrotransfected with the above vectors. Specifically, the electrotransfection was performed by the following steps: $1\times10^7$ T cells were taken and resuspended in 500 $\mu$L of electrotransfection buffer solution, and then thoroughly blew up and down to obtian a cell suspension; the cell suspension was then added with the above vector plasmids (10 ug), thoroughly blew up and down, and transferred into a sterile and clean electrotransfection cup; electric shock operation was performed twice under predetermined conditions with the setting parameters: 300 V, 10 ms; after the electric shock operation was completed, the electrotransfection cup was placed on ice for incubation for 10 min, so that nucleic acids fully entered into the cells; the electric shock cup was taken out from the ice, the cells were transferred from the electric shock cup, filtered, and then counted, seeded into fresh DMEM at a certain cell density, and incubated in a 5% $CO_2$ incubator at 37°C.

The target expression of T cells were detected by flow cytometry 2-3 days after electrotransfection. Knockout efficiency of a target gene = (expression level of control group-expression level of experimental group)/expression level of control group $\times100\%$. The knockout efficiency of PD-1, CTLA4, LAG-3 and Tim-3 genes of T cells was 98.5%, 98.7%, 99.2% and 97.2%, respectively.

(2) Production of retrovirus for CAR expression

A CD19-CAR sequence was constructed and then inserted into a lentivirus packaging system. After transfection of each plasmid into Phoenix ECO cell line (ATCC) using Lipofectamin 3000 (Invitrogen), culture supernatant containing ecotropic retrovirus secreted for 24-48 h was added to PG13 retroviral packaging cell line (ATCC) and spin infection (2500 rpm, 90 min) was performed.

The culture supernatant of the PG13 retrovirus-producing cell line prepared by the above method was harvested and filtered (0.45 $\mu$m filter) to remove residual cell particles, and then concentrated 4 times using a centrifugal filtration device (Millipore Amicon 100KD cut-off) to obtain CD19-CAR retrovirus, which was used as a retrovirus concentrate for construction of CAR-T cells.

(3) Preparation of CAR-Crispered-T cells

T cells with immune checkpoint gene knockout were added to a 24-well plate coated with anti-CD3 antibody (OKT3, 10 μg/ml, BioXcell) together with anti-CD28 antibody (CD28.2, 2 μg/ml, BD Biosciences), and cultured for 48 h to activate T cells. Activated T cells were washed twice and used for retrovirus transduction.

After overnight coating of a 24-well plate with Retronectin (20 μg/ml, TaKaRa) at 4°C, 2% BSA-DPBS was added to the 24-well plate washed, followed by blocking at 37°C for 30 min. After washing, 1 ml of the retrovirus concentrate was added, followed by centrifugation at 2000×g, 32°C for 2 h to adhere the retroviruses to the bottom of the wells. After removal of the retrovirus concentrate and washing of the wells, 1 ml of activated T cells ($1\times10^6$ cells/ml) were added to the wells, followed by centrifugation at 1000×g, 32°C for 10 min to adhere the cells to the retroviruses.

Subsequently, the cells were incubated for 48 h in the presence of human IL-2 (300 IU/ml, Proleukin, Novartis). In this way, retrovirus-transduced T cells were obtained, then the retrovirus-transduced T cells were washed twice, and then proliferated with a fresh culture medium containing human IL-2 (200 IU/ml) for 3 to 6 days to be used as CAR-Crispered-T cells.

With regard to the CAR protein expression on the cell surface, CAR-Crispered-T cells proliferated for 3 days after retrovirus transduction were stained with CD19-Ck protein (fusion protein of CD19 extracellular domain and human immunoglobulin κ chain constant region (Ck)) and anti-Ck-APC (BioLegend), and then detected by flow cytometry (FACS-Calibur, BD Biosciences).

According to the detection results, CD19-CAR-Crispered-T cells were successfully constructed.

According to the construction method described in Preparation Example 4, other tumor antigen-CAR-Crispered-T cells may be obtained by replacing CD19 with other types of tumor antigen. The types of tumor antigen may also be CD22, BCMA, MUC-1, cMet, Claude18.2, MSLN, EGFR, VEGFR2, HER-2, TPBG, etc. A person skilled in the art may construct other CAR-Crispered-T cells by the same method except for different CARs loaded on the T cells, and detect the success of construction by the same method.

When the tumor antigen was CD22, BCMA, MUC-1, cMet, Claude18.2, MSLN, EGFR, VEGFR2, HER-2 or TPBG respectively, CD22-CAR-Crispered-T cells, BCMA-CAR-Crispered-T cells, MUC-1-CAR-Crispered-T cells, cMet-CAR-Crispered-T cells, Claude18.2-CAR-Crispered-T cells, MSLN-CAR-Crispered-T cells, EGFR-CAR-Crispered-T cells, VEGFR2-CAR-Crispered-T cells, HER-2-CAR-Crispered-T cells, and TPBG-CAR-Crispered-T cells were obtained, respectively.

Preparation Example 5

Preparation Example 5 provides a method for constructing EGFR-targeted STAR-T cells. The method specifically included the following steps:

(1) Determination of sequences of TCR constant regions

The constant regions (C regions) of α and β chains of TCR in STAR were cloned from cDNA of human peripheral blood T cells by PCR. On the basis of the original TCR sequence, amino acid sites 48 and 57 of the constant regions of α and β chains were mutated to cysteine, respectively, to help form an additional disulfide bond (named E1-TCR) between α and β chains, increasing their pairing efficiency. The amino acid sequence of the TCRα chain constant region is shown in SEQ ID NO 67, and the amino acid sequence of the TCRβ chain constant region is shown in SEQ ID NO 68.

(2) Determination of sequences of EGFR-targeted antibody

The antibody heavy chain variable region (VH) and the antibody light chain variable region (VL) are selected from Cetuximab (Cetux), which is only used as an example to explain the present application, and may be substituted with other known antibodies.

(3) Construction of EGFR-targeted STAR

STAR contained two polypeptide chains, the Cetux-VL and TCR-β chains were fused to a first polypeptide chain, and Cetux-VH and TCR-α chains were fused to a second polypeptide chain. The gene sequences of STAR were linked by polypeptides furin and p2A at protein cleavage sites, the two polypeptide chains were transcribed and translated into a fusion polypeptide, and the fusion polypeptide was then cleaved by corresponding proteases of furin and p2A into two independent protein subunits. These two subunits are covalently bound by disulfide bonds and form complexes with

endogenous CD3 subunits ($\varepsilon$, $\delta$, $\gamma$, $\zeta$) of T cells.

The whole gene was inserted into lentiviral expression vector pHAGE via restriction endonuclease sites NheI and NotI. The vector carried ampicillin resistance, EF1 alpha promoter and IRES-RFP fluorescent reporter gene.

(4) Cloning and assembly of gene fragments

The four fragments "CetuxVL", "TCR$\beta$-C", "Cetux-VH" and "TCR$\alpha$-C" were cloned from pHAGE-Cetux-28zCAR vector and pHAGE-E1-TCR vector respectively. Each primer pair contained homologous 25bp base pairs. The four fragments were recombined and ligated into lentiviral vectors by Gibson Assembly in one step, thus obtaining STAR.

The amino acid sequence of the Cetux VL is shown in SEQ ID NO 69, and the amino acid sequence of the Cetux VL is shown in SEQ ID NO 70.

(5) Vector transformation and sequencing

The product from Gibson Assembly was transformed into DH5$\alpha$ strain and allowed to grow overnight on LB plates containing ampicillin. Monoclones were selected for sequencing. Sequencing primers were seq-pHAGE-F and seq-pHAGE-R on pHAGE vector.

(6) Plasmid extraction

The monoclones with correct sequencing results were seeded in LB liquid medium and incubated overnight. Plasmids were extracted using a kit with endotoxin removal function. Plasmid concentration was measured by Nanodrop. The final plasmid concentration was about 1000 ng/$\mu$l, and the A260/A280 ratio was greater than 1.8.

(7) Lentivirus packaging

The pHAGE vector carrying the target gene, together with the packaging plasmids pMD2.G and psPAX2, was transfected (using PEI) into 293T cells in proportion. Supernatants of cell culture medium at 48 h and 72 h were collected, mixed with PEG8000, allowed to stand overnight and centrifuged to obtain virus pellets. Virus pellets were resuspended in a small volume of medium to concentrate the virus.

(8) Isolation, culture and lentivirus infection of human primary T cells

CD4 and CD8 T cells were purified from human peripheral blood cells by using a whole T cell magnetic beads separation kit. T cells were stimulated and activated in a culture dish coated with anti-CD3/CD28 antibody for 48 h to 72 h, and it was observed that T cells are increased in size, aggregated and polarized in shape. In this case, the target gene was transferred into T cells using a lentivirus vector, and the infection method was centrifugation at 1500 rpm, 32°C for 2 h. After virus infection, the cells were cultured in RPMI1640 medium containing 20% serum and 200 IU IL-2 to a sufficient quantity.

(9) Detection of coating and antigen binding ability of EGFR-targeted STAR

T cells infected for 3 days were stained with an anti-human TCR $\alpha$/$\beta$-BV421 flow antibody, and then flow cytometry was performed. The results showed that STAR could be stained by the anti-TCR $\alpha$/$\beta$ antibody compared to non-transgenic negative control cells and its staining level was comparable to that of native E1-TCR. This result indicates that STAR molecules can be coated and that its $\alpha$ and $\beta$ chains can be paired.

T cells infected for three days were stained with flow antibodies against EGFR-His and anti-His-APC antigen proteins, and then flow cytometry was performed. The assay results showed that STAR showed better staining performance than negative control cells of native E1-TCR (not specific for EGFR) and its staining level was comparable to that of anti-EGFR CAR. These results suggest that STAR has antigen recognition and binding abilities comparable to CAR molecules.

(10) Co-incubation of EGFR-targeted STAR-T cells with target cells and detection of their ability to mediate T cell activation

T cells infected for three days were cultured in EGFR antigen-coated cell culture plates and co-incubated with tumor cells A549 (EGFR-positive human lung cancer cell line). 24 h later, cells were harvested and stained with anti-human CD69-FITC flow antibody, and then flow cytometry was performed. The results showed that STAR could induce T cells to express CD69 activation marker under antigen stimulation. That is, STAR could mediate T cell activation after antigen stimulation, and the activation degree thereof was similar to that of CAR. Moreover, STAR has no self-activation phenomenon in resting state without antigen stimulation, but CAR has higher self-activation level.

According to the construction method described in Preparation Example 5, other tumor antigen-STAR-T cells may be obtained by replacing CD19 with other types of tumor antigen. The types of tumor antigen may also be CD22, BCMA, MUC-1, cMet, Claude18.2, MSLN, EGFR, VEGFR2, HER-2, TPBG, etc. A person skilled in the art may construct other STAR-T cells by the same method except for different STARs loaded on the T cells, and detect the success of construction by the same method.

When the tumor antigen was CD22, BCMA, MUC-1, cMet, Claude18.2, MSLN, EGFR, VEGFR2, HER-2 or TPBG respectively, CD22-STAR-T cells, BCMA-STAR-T cells, MUC-1-STAR-T cells, cMet-STAR-T cells, Claude18.2-STAR-T cells, MSLN-STAR-T cells, EGFR-STAR-T cells, VEGFR2-STAR-T cells, HER-2-STAR-T cells, and TPBG-STAR-T cells were obtained, respectively.

Preparation Example 6

Preparation Example 6 provides a method for constructing CAR-NK cells. The method specifically included the following steps:

(1) Preparation of NK cells

Peripheral blood mononuclear cells (PBMC) were obtained by density gradient centrifugation.

Specifically, the density gradient centrifugation was performed by the following steps: 20 mL of human peripheral blood was drawn into a centrifuge tube using a blood collection tube containing anticoagulant and centrifuged at 2000 rpm for 10min; upper plasma was collected, the remaining blood cell pellets were resuspended in an equal volume of pre-warmed normal saline, and mixed well; in another centrifuge tube, the mixed blood cell pellets were gently added to the surface of lymphocyte separation medium according to a volume ratio of 1:1, followed by centrifugation at 18000 rpm for 25 min; a white lymphocyte layer was carefully pipetted; a white membrane layer was transferred into a new centrifuge tube and supplemented with PBS to 45 mL, then centrifuged at 1500 rpm for 5 min, and washed twice; the cells were suspended in an appropriate amount of RPMI1640 (containing 10% FBS) complete medium and counted.

NK cells were sorted out using an NK cell sorting kit (purchased from Biolegend), expanded culture was performed using RPMI1640 (containing 10% FBS), and cytokines IL-18 (100U/mL) and IL-15(100U/mL) were added for activation.

(2) Preparation of CAR-NK cells

The chimeric antigen receptor gene fragment (CD19) of the present invention was introduced into psb1576 vector, and then the vector was introduced into competent cells. The plasmid was extracted with a plasmid extraction kit (purchased from Axygen), and sequencing was performed to identify whether the target gene sequence was correct. The positive clone plasmids were handed over to CRO Company to synthesize lentiviral vectors respectively.

The prepared NK cells were activated and added to a fresh culture medium, incubated at 37°C, 5% $CO_2$ for 10 days, centrifuged and collected. The cells were resuspended in fresh culture medium and the cell density was adjusted to $1 \times 10^6$ cells/mL; lentivirus was added to 24-well culture plates (MOI=10), well mixed, and incubated in a 5 % $CO_2$ incubator at 37°C for 48-96 h, and then the culture medium was changed with fresh one. The transduction efficiency of CAR-NK cells (CD19-CAR-NK cells) was determined by PCR, and the positive rate was over 95%, which met the experimental requirements.

According to the construction method described in Preparation Example 6, other tumor antigen-CAR-NK cells may be obtained by replacing CD19 with other types of tumor antigen. The types of tumor antigen may also be CD22, BCMA, MUC-1, cMet, Claude18.2, MSLN, EGFR, VEGFR2, HER-2, TPBG, etc. A person skilled in the art may construct other CAR-NK cells by the same method except for different CARs loaded on the T cells, and detect the success of construction by the same method.

When the tumor antigen was CD22, BCMA, MUC-1, cMet, Claude18.2, MSLN, EGFR, VEGFR2, HER-2 or TPBG respectively, CD22-CAR-NK cells, BCMA-CAR-NK cells, MUC-1-CAR-NK cells, cMet-CAR-NK cells, Claude18.2-CAR-NK cells, MSLN-CAR-NK cells, EGFR-CAR-NK cells, VEGFR2-CAR-NK cells, HER-2-CAR-NK cells, and TPBG-CAR-NK cells were obtained, respectively.

Preparation Example 7

Preparation 7 provides a method for constructing IPSC CAR-NK cells. For the construction method in this preparation example, reference is made to the construction method disclosed in CN114958771A. The method specifically included the following steps:

S1: IPSCs obtained by reprogramming blood cells were infected with a lentivirus vector carrying chimeric antigen receptor (CAR) gene (CD19) to obtain IPSC cells expressing the chimeric antigen receptor CAR;

S2: the IPSCs expressing the CAR were cultured in a culture medium containing 55 ng/ml stem cell factor SCF, 2 $\mu$m/ml GSK3$\beta$ inhibitor, 28ng/ml bone morphogenetic protein (BMP), 55 ng/ml FGF2 and 35 mg/ml VEGF, and 30 ng/ml FLT-3L ligand, 60 ng/ml thrombopoietin TPO and 20ng/ml IL7 were added for directional differentiation induction to obtain hematopoietic progenitor cells (HPC);

S3: the HPCs were transferred into a serum-free DMEM medium containing 1.0% P/S dual antibody, 60 ng/ml TGF-$\beta$ transforming growth factor, 15 ng/ml cytokines, 5 $\mu$M phosphatidylinositol 3-kinase inhibitor and 4 $\mu$M antagonists, where the antagonists were 1 $\mu$M angiotensin II type 1 receptor AT1 antagonist (sartan), 1 $\mu$M Mek/Erk antagonist (PD0325901) and 1 $\mu$M TGF$\beta$ antagonist (SB431542); cytokines were a combination of 3 ng/ml SCF, 4 ng/ml TPO, 3 ng/ml IL3, 3 ng/ml IL-11 and 2 ng/ml IL-15; the culture medium was replaced every 18 hours, and the cells were cultured for 5 days;

The cell density was adjusted to $2\times10^2$ cells/mL, and the cells were then sequentially transferred to a first amplification medium containing 0.2 g/L tranexamic acid and a second amplification medium containing 0.8 g/L tranexamic acid for amplification, where the first amplification medium was DMEM containing 6% (v/v) inactivated autoserum, 100 ng/mL IL-2, 3 ng/mL FGF2, 0.008 g/L i-inositol, 0.04 g/L folic acid, 0.15 g/L sheep placentin, 0.7 g/L pyridoxine hydrochloride and 0.2 g/L tranexamic acid; the same amplification medium was replaced after 24 hours.

After 48 hours of culture in the first amplification medium, the cells were transferred to the second amplification medium, which was DMEM containing 2.5% (v/v) inactivated autoserum, 110 ng/mL IL-2, 4 ng/mL FGF2, 0.013 g/L i-inositol, 0.04 g/L folic acid, 0.2 g/L sheep placentin, 1.3 g/L pyridoxine hydrochloride and 0.8 g/L tranexamic acid; the culture was continued to 72 hours to obtain antigen-specific IPSC-CAR-NK cells (CD19-IPSC CAR-NK cells).

According to the construction method described in Preparation Example 7, other tumor antigen-IPSC CAR-NK cells may be obtained by replacing CD19 with other types of tumor antigen. The types of tumor antigen may also be CD22, BCMA, MUC-1, cMet, Claude18.2, MSLN, EGFR, VEGFR2, HER-2, TPBG, etc. A person skilled in the art may construct other IPSC CAR-NK cells by the same method except for different CARs loaded on the T cells, and detect the success of construction by the same method.

When the tumor antigen was CD22, BCMA, MUC-1, cMet, Claude18.2, MSLN, EGFR, VEGFR2, HER-2 or TPBG respectively, CD22-IPSC CAR-NK cells, BCMA-IPSC CAR-NK cells, MUC-1-IPSC CAR-NK cells, cMet-IPSC CAR-NK cells, Claude18.2-IPSC CAR-NK cells, MSLN-IPSC CAR-NK cells, EGFR-IPSC CAR-NK cells, VEGFR2-IPSC CAR-NK cells, HER-2-IPSC CAR-NK cells, and TPBG-IPSC CAR-NK cells were obtained, respectively.

Preparation Example 8

Preparation Example 8 provides a method for constructing CAR-M cells. The method specifically included the following steps:

1) Construction of PD-L1-CAR lentivirus vector and preparation of PD-L1-CAR lentivirus

(1) Construction of PD-L1-CAR lentivirus vector

The PD-L1-CAR sequence (SEQ ID NO 71) was designed and synthesized by screening and investigation in previous experiments: from 5 'end to 3' end, there were signal peptide SP, PD-L1scFv antibody, transmembrane region of CD8 $\alpha$, intracellular functional region of CD86 and intracellular functional region of FcγRI. The synthesized PD-L1-CAR sequence was cloned into pLVX-EF1$\alpha$-AcGFP1-N1 vector to obtain PD-L1-CAR lentivirus vector.

(2) Preparation of PD-L1-CAR lentivirus

The PD-L1-CAR lentivirus vector and lentivirus packaging vectors pMD2.G and psPAX2 were was co-transfected into 293T cells to prepare PD-L1-CAR lentivirus. The lentivirus was packaged when the fusion degree of 293T cells was 70%-80%. The virus supernatant was collected at 24h and 48h after lentivirus packaging, centrifuged at 4000 rpm for 10 min, filtered with 0.45 um filter membrane to remove cell debris, then ultra-high-speed centrifuged at 25000 rpm for 2h at 4°C to obtain PD-L1-CAR lentivirus concentrate. The PD-L1-CAR lentivirus concentrate was subpackaged and stored at -80°C.

(3) Identification of PD-L1-CAR lentiviral vector

After the PD-L1-CAR lentivirus expression plasmid was cleaved with restriction enzymes EcoRI and BamHI and then detected by electrophoresis for 40 min on 1.2% agarose gel at 120V, and the electrophoretic bands were analyzed to determine whether the recombinant PD-L1-CAR lentivirus expression plasmid was correctly inserted into the PD-L1 gene fragment.

2) Expansion culture of hematopoietic stem cells derived from human umbilical cord blood

Preparation of hematopoietic stem cell complete medium: Hematopoietic cell serum-free medium StemSpanTM SFEM (StemCell, 09600) was supplemented with cytokines SCF (100 ng/ml), Flt-3L (100ng/ml), TPO (50ng/ml), IL-6 (50ng/ml), LDL (10ug/ml), SR1 (750nM) and UM171 (35nM).

On day 1, PBMCs were isolated from umbilical cord blood and resuspended in the prepared hematopoietic stem cell complete medium at a cell density of $5 \times 10^6$ cells/ml; 10 mL of PBMCs were seeded into T25 cell culture flasks; the T25 cell culture flasks were placed vertically on a shaker and shaken at 100 rpm/min for incubation at 37°C, 5% $CO_2$ in a carbon dioxide incubator. On day 2-5, 2-3 mL of fresh CD34$^+$ hematopoietic stem cell complete medium every day; when the culture volume of each culture flask reached 20ml-25ml, the rotation speed of the shaker was adjusted to 110-120 rpm/min. On days 6-9, 4-6mL of fresh CD34$^+$ hematopoietic stem cell complete medium was added every day. On day 9, samples were taken for counting and the proportion of CD34$^+$ hematopoietic stem cells (HSCs) was detected by flow cytometry.

3) Preparation of PD-L1-CAR-HSCs

Infection of HSCs with PD-L1-CAR lentivirus: HSCs were infected with PD-L1-CAR lentivirus (MOI=60), and treated with puromycin (0.25 µg/ml) for 3 days at 48 h after infection to screen HSCs stably expressing PD-L1-CAR. A stable PD-L1-CAR-HSCs cell line was constructed. After expansion in a large quantity, the PD-L1-CAR-HSCs were frozen in liquid nitrogen for future use.

The infection efficiency of PD-L1-CAR lentivirus on HSCs was indirectly detected by detecting the proportion of GFP+PD-L1-CAR HSCs.

4) Induced differentiation of PD-L1-CAR-HSCs into PD-L1-CAR-M

Preparation of macrophage differentiation medium M1: RPMI 1640 medium was supplemented with 2% (v/v) B27 additive, 2mM Glutamax, 1% (v/v) nonessential amino acids (NEAA), 50 ng/ml vitamin C, 10 ng/ml human interleukin-3 (IL-3) and 50 ng/ml human macrophage colony stimulating factor (M-CSF).

Preparation of macrophage differentiation medium M2: RPMI 1640 medium was supplemented with 2% (v/v) B27 additive, 2mM Glutamax L-alanyl-L-glutamine, 1% (v/v) nonessential amino acids (NEAA), 50 ng/ml vitamin C and 50 ng/ml human M-CSF.

PD-L1-CAR-HSCs from step 3 were resuspended in M1 and incubated in a 5% $CO_2$ incubator at 37°C for 5 days, and half of the M1 medium was replaced every other day. After 5 days, the cells were collected, centrifuged, resuspended in M2, and incubated in a 5% $CO_2$ incubator at 37 °C for 3 days to obtain CAR-macrophages derived from human hematopoietic stem cells (PD-L1-CAR-M).

5) Identification of PD-L1-CAR-M cells

PD-L1-CAR-M cells obtained by induced differentiation were collected, washed and resuspended in PBS containing 0.1% BSA, and incubated with CD14-PE (399204, Biolegend) and CD11b-FITC (301330, Biolegend) antibodies for 20 min at room temperature in the dark. The expression of CD14 and CD11b on the surface of PD-L1-CAR-M cells was detected and analyzed by flow cytometry. It was proved that CAR-macrophages derived from human HSCs through induced differentiation were successfully obtained.

According to the construction method described in Preparation Example 8, other tumor antigen-CAR-M cells may be obtained by replacing PD-L1 with other types of tumor antigen. The types of tumor antigen may also be CD19, CD22, BCMA, MUC-1, cMet, Claude18.2, MSLN, EGFR, VEGFR2, HER-2, TPBG, etc. A person skilled in the art may construct other CAR-M cells by the same method except for different CARs loaded on the Tcells, and detect the success of construction by the same method.

When the tumor antigen was CD19, CD22, BCMA, MUC-1, cMet, Claude18.2, MSLN, EGFR, VEGFR2, HER-2 or TPBG respectively, CD19-CAR-NK cells, CD22-CAR-NK cells, BCMA-CAR-NK cells, MUC-1-CAR-NK cells, cMet-CAR-NK cells, Claude18.2-CAR-NK cells, MSLN-CAR-NK cells, EGFR-CAR-NK cells, VEGFR2-CAR-NK cells, HER-2-CAR-NK cells, and TPBG-CAR-NK cells were obtained, respectively.

Preparation Examples 9-12

Preparation Examples 9-12 respectively provide a method for treating tumors using a combination of an oncolytic virus vaccine and immune cells.

According to the method, tumors are treated using a combination of an oncolytic virus vaccine and immune cells. The oncolytic virus vaccine includes a recombinant oncolytic virus expressing a tumor antigen, and is used for targeting tumor cells; the immune cells express chimeric antigen receptors.

The recombinant oncolytic virus includes an M protein, a G protein, an N protein, a P protein, an L protein and a tumor antigen. The M protein, the G protein, the N protein, the P protein, and the L protein were all obtained by preforming site-directed mutagenesis on the wild-type VSV virus Indiana MuddSummer subtype, and the tumor antigen was obtained by inserting a gene encoding the tumor antigen.

The preparation examples 9-12 differ in the mutated sites of the M protein. mutated sites of the M protein were amino acid sequences as shown in SEQ ID NO 3, SEQ ID NO 9, SEQ ID NO 10 and SEQ ID NO 11 respectively. The G protein included an amino acid sequence as shown in SEQ ID NO 13; the N protein included an amino acid sequence as shown in SEQ ID NO 15; the P protein included an amino acid sequence as shown in SEQ ID NO 17; and the L protein included an amino acid sequence as shown in SEQ ID NO 19. Mutated sites of the proteins and types of tumor antigens are shown in Table 1.

I. A construction method for the recombinant oncolytic virus provided by said preparation examples was as follows:

(1) Construction of vector

Using a pRV-core plasmid (BioVector NTCC (National Type Culture Collection)) as a template, the mutated sites of the M protein, the mutated sites of the G protein, the mutated sites of the N protein, the mutated sites of the P protein and the mutated sites of the L protein shown in Table 1 were introduced by PCR.

Gene fragments containing XbaI and MluI restriction sites and including the protein mutated sites described above was synthesized respectively. PCR amplification was performed using the gene fragments as templates. The PCR products were subjected to 1% agarose gel electrophoresis and double-digested with XbaI and MluI, and a gene fragment with the mutated sites of the M protein, a gene fragment with the mutated sites of the G protein, a gene fragment with the mutated sites of the N protein, a gene fragments with the mutated sites of the P protein, a gene fragment with the mutated sites of the L protein were obtained by gel extraction using a gel extraction kit. The pRV-core plasmid was double-digested with XbaI and Mlu I, and a pRV-core restriction digestion extraction skeleton fragment was obtained by gel extraction using a gel extraction kit.

The gene fragment with the mutated sites of the M protein, the gene fragment with the mutated sites of the G protein, the gene fragment with the mutated sites of the N protein, the gene fragments with the mutated sites of the P protein and the gene fragment with the mutated sites of the L protein were respectively ligated to the pRV-core restriction digestion extraction skeleton fragment for transformation, the transformation product was then plated, and monoclones were selected and shaken for identification by PCR. The plasmid was extracted to obtain the constructed plasmid pRV-core Mut, which was sequenced by a sequencing company.

(2) Insertion of exogenous gene

The plasmid pRV-core Mut obtained in step (1) was double-digested with Xho I and Mlu I to extract a long fragment.

An exogenous gene encoding the tumor antigen was synthesized by a gene synthesis company and amplified with corresponding primers. The amplification product was double-digested with Xho I and Nhe I and a target gene fragment was extracted. The double-digested pRV-core Mut and the exogenous gene fragment were ligated and transformed. Monoclones were selected and identified by PCR or enzyme digestion, and then sequenced by a sequencing company to obtain a plasmid pRV-core Mut carrying the exogenous gene.

Table 1 Recombinant oncolytic viruses and immune cells in Preparation Examples 9-12

| Prepara tion Exa mpl e | Recombinant oncolytic virus | | | | | | | | | | | | | | | | | Immune cell | |
|---|---|---|---|---|---|---|---|---|---|---|---|---|---|---|---|---|---|---|---|
| | M protein | | | G protein | | | N protein | | | P protein | | | L protein | | | Tumor antigen | | | |
| | Mutated sites and changes before and after | Numb er of mut | Se qu en ce | Mutated sites and changes before and after | N u m b e | Se qu en ce | Mutated sites and changes before and after | N u m b e | Se qu en ce | Mutated sites and changes before and | N u m b e | Se qu en ce | Mutated sites and changes before and | N u m b e | Se qu en ce | Ty pe | Se qu en ce | Type | Seq uenc e of anti gen |

| mutation | ations | | mutations | r of mutations | | mutations | r of mutations | | after mutations | r of mutations | | after mutations | r of mutations | | | | | receptor |
|---|---|---|---|---|---|---|---|---|---|---|---|---|---|---|---|---|---|---|
| 9 — N32S, N49D, M51R, H54Y, V221F, 225I, S226R | 7 | SEQ ID NO 3 | | | | | | | | | | | | | | | | |
| 10 — N32S, M33A, N49D, M51R, H54Y, 221F, V225I, S226R | 8 | SEQ ID NO 9 | V53I, A141V, D172Y, K217E, D232G, V331A, V371E, G436D, T438S, F453L, T471I, Y487H | 12 | SEQ ID NO 13 | I14V, R155K, S353N | 3 | SEQ ID NO 15 | R50K, V76A, D99E, L126S, L140S, H151Y, I168M, K170E, Y189S, N237D | 10 | SEQ ID NO 17 | S87P, I487T | 2 | SEQ ID NO 19 | CD19 | SEQ ID NO 20 | CD19-CAR-T cell | SEQ ID NO 37 |
| 11 — N32S, M33A, N49D, M51R, H54Y, 133T, V221F, 225I, S226R | 9 | SEQ ID NO 10 | | | | | | | | | | | | | | | | |
| 12 — N32S, N49D, M51R, H54Y, A133T, 221F, V225I, S226R | 8 / 11 | SEQ ID NO | | | | | | | | | | | | | | | | |

(3) Virus rescue

The plasmid pRV-core Mut carrying the exogenous gene was transfected into a BSR-T7 cell (purchased from ATCC, American Type Culture Collection) by cell transfection technology using a calcium phosphate transfection kit (Thermo Fisher Scientific).

Four plasmids, i.e., the pRV-core Mut, pP, pN, and pL, were mixed at a ratio of 10:5:4:1, and the total amount of the plasmids was 5 $\mu$g; the plasmid mixture was then diluted with 200 $\mu$l of an Opti-MEM (Thermo Fisher Scientific), and 7.5 $\mu$l of a transfection reagent Plus Reagent (Life Technologies) was added to obtain a transfection plasmid premix, where the pP was a plasmid carrying the phosphoprotein genes of a rhabdovirus, the pN was a plasmid carrying the nucleoprotein genes of a rhabdovirus, and the pL was a plasmid carrying the polymerase protein genes of a rhabdovirus; parent vectors corresponding to the three plasmids pN, pP, and pL were all pCAGGS (purchased from ATCC).

10 $\mu$l of Lipofectamine LTX (Thermo Fisher Scientific) was diluted with 200 $\mu$l of the Opti-MEM to obtain an LTX mixed solution.

Plasmid transfection was performed according to the method described in the instruction manual of Lipofectamine LTX. Six hours later, the BSR-T7 cell was washed twice with PBS and then seeded in DMEM (Thermo Fisher Scientific) supplemented with 10% fetal bovine serum and incubated for three days.

The cell supernatant of the BSR-T7 cell culture solution was transferred to a Vero cell (Thermo Fisher Scientific), and the Vero cell was incubated at 37 °C for three days. The cells were observed for green fluorescence under a fluorescence microscope to determine the result of virus rescue. The rescued mutant rhabdovirus library was further passaged via the Vero cell, and monoclonal virus strains were selected in an established plaque screening system.

(4) Gene sequencing. The viral genomic RNA was extracted using a Trizol kit, and reverse transcription was performed using random primers. The reverse transcribed cDNA was subjected to PCR using primers designed for the gene sequence of the M protein, primers designed for the gene sequence of the G protein, primers designed for the gene sequence of the N protein, primers designed for the gene sequence of the P protein, primers designed for the gene sequence of the L protein and primers designed for the gene sequence encoding the tumor antigen.

The primer sequences designed for the gene sequence of the M protein were:

□ PF: ATGAGTTCCTTAAAGAA;
□ PR: TCATTTGAAGTGG.

The primer sequences designed for the gene sequence of the G protein were:

□ PF: ATGAAGTGCCTTTTGTACTTAG;
□ PR: TTACTTTCCAAGTCGGTTCATCT.

The primer sequences designed for the gene sequence of the N protein were:

□ PF: ATGTCTGTTACAGTCAAGAG;
□ PR: TCATTTGTCAAATTCTGACTT.

The primer sequences designed for the gene sequence of the P protein were:

□ PF: ATGGATAATCTCACAAAAGTTCG;
□ PR: CTACAGAGAATATTTGACTCTCG.

The primer sequences designed for the gene sequence of the L protein were:

PF: ATGGAAGTCCACGATTTTGAGA;
PR: TTAATCTCTCCAAGAGTTTTCCT.

The primer sequences designed for the gene sequence encoding the tumor antigen were:

CD19 F: ACGCTCGAGATGCCACCTCCTCGCCTCC;
CD19 R: TCTGGCTAGCTCATCTTTTCCTCCTCAGG.

The product was extracted after 1% agarose gel electrophoresis and sequenced by a sequencing company. The sequencing results are shown in Table 1.

II. A construction method for the oncolytic virus vaccine provided by each of said preparation examples was as follows:

(1) The packaging process of the recombinant oncolytic virus described above. Specifically, the packaging process included the following steps:

1) Infection and inoculation of BSR-T7 cell (purchased from ATCC) with poxvirus vTF7-3 (BioVector NTCC) expressing T7 RNA polymerase

Specifically, the BSR-T7 cell was plated on a 6-well plate, with $3 \times 10^5$ cells/well; 14-16 hours after plating, the poxvirus vTF7-3 expressing the T7 RNA polymerase was added to infect the BSR-T7 cell; 6 hours after infection, the BSR-T7 cell was rinsed once with Dulbecco's Phosphate Buffered Saline (DPBS, Thermo Fisher Scientific) for transfection later.

2) Transfection process

Specifically, the transfection process included the following steps: four plasmids, the pRV-core Mut, pP, pN, and pL, were mixed at a ratio of 10:5:4:1, and the total amount of the plasmids was 5 µg; the plasmid mixture was then diluted with 200 µl of an Opti-MEM (Thermo Fisher Scientific), and 7.5 µl of a transfection reagent Plus Reagent (Life Technologies) was added to obtain a transfection plasmid premix, where the pP was a plasmid carrying the phosphoprotein genes of a rhabdovirus, the pN was a plasmid carrying the nucleoprotein genes of a rhabdovirus, and the pL was a plasmid carrying the polymerase protein genes of a rhabdovirus; parent vectors corresponding to the three plasmids pN, pP, and pL were all pCAGGS (purchased from ATCC).

10 µl of Lipofectamine LTX (Thermo Fisher Scientific) was diluted with 200 µl of the Opti-MEM to obtain an LTX mixed solution.

200 µl of the LTX mixture was mixed with 200 µl of the transfection plasmid premix, followed by incubation at room temperature for 15 min, to obtain an LTX-DNA mixed solution.

The DPBS in the 6-well plate in step 1) was replaced with the Opti-MEM, the LTX-DNA mixed solution was dropwise added to the 6-well plate in which the BSR-T7 cell was incubated, and the 6-well plate was then shaken gently to evenly distribute the LTX-DNA mixed solution in the 6-well plate; 6-8 hours after transfection, the transfection reagent was removed and 3 ml of fresh complete medium (Thermo Fisher Scientific) was then added; 72 hours later, the cell supernatant of the BSR-T7 cell was harvested and filtered with a 0.22 µm filter to obtain the recombinant oncolytic viruses, i.e., the oncolytic virus vaccines, respectively corresponding to the preparation examples.

IV. Immune cells

Immune cells were immune cells prepared by the construction method provided in Preparation Example 1.

Preparation Examples 13-29

Preparation Examples 13-29 respectively provide a method for treating tumors using a combination of an oncolytic virus vaccine and immune cells.

According to the method, tumors are treated using a combination of an oncolytic virus vaccine and immune cells. The oncolytic virus vaccine includes a recombinant oncolytic virus expressing a tumor antigen, and is used for targeting tumor cells. Immune cells and antigen receptors expressed thereby are shown in Table 2.

The recombinant oncolytic virus includes an M protein, a G protein, an N protein, a P protein, an L protein and a tumor antigen. The mutated sites of the M protein, the G protein, the N protein, the P protein and the L protein are the same as the corresponding mutated sites in Preparation Example 9, and the difference lies in the types of the tumor antigen and the details are shown in Table 2.

I. The construction method of the recombinant oncolytic viruses provided in said preparation examples is the same as the construction method in Preparation Example 9, except that:

the tumor antigens as shown in Table 2 were introduced by PCR during insertion of exogenous gene in step (2); and

step (4) includes sequencing of each type of tumor antigen. The viral genomic RNA was extracted using a Trizol kit, and reverse transcription was performed using random primers. The reverse transcribed cDNA was subjected to PCR using primers designed for gene sequence encoding the tumor antigen.

The primer sequences designed for the gene sequence encoding the antigen were:

1) CD22 F: ATGCATCTCCTCGGCCCCT;
CD22 R: TCAGAGCCCACAGATTGCCAGG.
2) BCMA F: ACGCTCGAGATGTTGCAGATGGCTGGGC;
BCMA R: TCTGGCTAGCTTATAGCAAAAACATTAGC.
3) MUC-1 F: ACGCTCGAGATGTCTGGTCATGCAAGC;

MUC-1-R: TCTGGCTAGCTTACAAGGCAATGAGATAG.
4) NY-ESO-1 F: ACGCTCGAGATGCAGGCAGAAGGAAG;
NY-ESO-1 R: TCTGGCTAGCTCATCTTCTCTGTCCGCTA.
5) MAGE A4 F: ACGCTCGAGACAGAGGAGCACCAAGGAG;
MAGE A4 R: TCTGGCTAGCATAGACTGAGGCATAAGGC.
6) cMet F: ACGCTCGAGATGGAGTGCAAGGAGGCC;
cMet R: TCTGGCTAGCTTACAGCCACAGGAAGAAG.
7) Claude 18.2 F: ACGCTCGAGATGGACCAGTGGAGCACCC;
Claude 18.2 R: TCTGGCTAGCTTAGGCGATGCACATCATC.
8) MSLN F: ACGCTCGAGATGGAAGTGGAGAAGACAG;
MSLN R: TCTGGCTAGCTCAGGCCAGGGTGGAGGCT.
9) EGFR F: ACGCTCGAGATGCGACCCTCCGGGACGG;
EGFR R: TCTGGCTAGCTTACATGAAGAGGCCGAT.
10) VEGFR2 F: ACGCTCGAGATGCAGAGCAAGGTGCTG;
VEGFR2 R: TCTGGCTAGCTCAGATGATGACAAGAAGT.
11) HER-2 F: ATGGAGCTGGCGGCCTTGTGCC;
HER-2 R: TTAGATGAGGATCCCAAAGACCA.
12) TPBG F: ATGTCTTCTCCCACCTCCTCGGCAT;
TPBG R: TCACAAATACAAAACCAGGAGGAAA.

The product was extracted after 1% agarose gel electrophoresis and sequenced by a sequencing company. The sequencing results are shown in Table 3.

II. Immune cells

Immune cells were immune cells prepared by the construction method provided in Preparation Examples 1-8.

Table 2 Recombinant oncolytic viruses and immune cells in Preparation Examples 9 and 13-29

| Preparation Example | M protein | | Tumor antigen | | Immune cell | |
|---|---|---|---|---|---|---|
| | Mutated sites and amino acids after mutation | | Type | Sequence | Type | Sequence of antigen receptor |
| 9 | | | CD19 | SEQ ID NO 20 | CD19-CAR-T cell | SEQ ID NO 37 |
| 13 | | | CD22 | SEQ ID NO 21 | CD22-CAR-T cell | SEQ ID NO 38, SEQ ID NO 39 |
| 14 | | | BCMA | SEQ ID NO 22 | BCMA-CAR-T cell | SEQ ID NO 40, SEQ ID NO 41 |
| 15 | | | NY-ESO-1 | SEQ ID NO 24 | NY-ESO-1 TCR-T cell | SEQ ID NO 58, SEQ ID NO 59 |
| 16 | | | MAGE A4 | SEQ ID NO 25 | MAGE A4 TCR-T cell | SEQ ID NO 60, SEQ ID NO 61 |
| 17 | M protein: N32S, N49D, M51R, H54Y, V221F, V225I, S226R. | | cMet | SEQ ID NO 26 | cMet -CAR-T cell | SEQ ID NO 42, SEQ ID NO 43 |
| 18 | G protein: V531, A141V, D172Y, K217E, D232G, V331A, V371E, G436D, T438S, F453L, T471I, Y487H. | | Claude 18.2 | SEQ ID NO 27 | Claude 18.2-CAR-T cell | SEQ ID NO 44, SEQ ID NO 45 |

(continued)

| Preparation Example | M protein | | Tumor antigen | | Immune cell | |
|---|---|---|---|---|---|---|
| | Mutated sites and amino acids after mutation | Type | Sequence | Type | Sequence of antigen receptor |
| 19 | N protein: I14V, R155K, S353N。 | MSLN | SEQ ID NO 28 | MSLN-CAR-T cell | SEQ ID NO 46, SEQ ID NO 47 |
| 20 | P protein: R50K, V76A, D99E, L126S, L140S, H151Y, I168M, K170E, Y189S, N237D. | EGFR | SEQ ID NO 29 | EGFR-CAR-T cell | SEQ ID NO 48, SEQ ID NO 49 |
| 21 | L Protein: S87P, 1487T. | VEGFR2 | SEQ ID NO 30 | VEGFR2-CAR-T cell | SEQ ID NO 50, SEQ ID NO 51 |
| 22 | | HER-2 | SEQ ID NO 31 | HER-2-CAR-T cell | SEQ ID NO 52, SEQ ID NO 53 |
| 23 | | TPBG | SEQ ID NO 32 | TPBG-CAR-T cell | SEQ ID NO 54, SEQ ID NO 55 |
| 24 | | CD19 | SEQ ID NO 20 | CD19-CAR-$\gamma\delta$-T cell | SEQ ID NO 37 |
| 25 | | CD19 | SEQ ID NO 20 | CD 19-CAR-Crispered-T cell | SEQ ID NO 37 |
| 26 | | EGFR | SEQ ID NO 29 | STAR-T-EGFR cell | SEQ ID NO 48, SEQ ID NO 49 |
| 27 | | CD19 | SEQ ID NO 20 | CD19-CAR-NK cell | SEQ ID NO 37 |
| 28 | | CD19 | SEQ ID NO 20 | CD19-IPSC CAR-NK cell | SEQ ID NO 37 |
| 29 | | CD19 | SEQ ID NO 20 | CD19-CAR-M cell | SEQ ID NO 37 |

Preparation Examples 30-83

Preparation Examples 30-83 respectively provide a method for treating tumors using a combination of an oncolytic virus vaccine and immune cells.

The method involves an oncolytic virus vaccine and immune cells. The oncolytic virus vaccine includes a recombinant oncolytic virus expressing a tumor antigen, and is used for targeting tumor cells; the immune cells express chimeric antigen receptors.

Said preparation examples 30-83 are different from Preparation Examples 13-29 in that the recombinant oncolytic virus further includes a cytokine, in addition to the M protein, the G protein, the N protein, the P protein, the L protein and the tumor antigen. The mutated sites of the M protein, the G protein, the N protein, the P protein and the L protein are the same as the corresponding mutated sites in Preparation Example 9, and the difference lies in the types of the tumor antigen and the types of the cytokine, and the details are shown in Table 3.

Specifically:

The cytokine included in the recombinant oncolytic virus provided in each of Preparation Examples 30-47 was IL-12 including amino acid sequences as shown in SEQ ID NOs 33 and 34. See Table 3 for details.

The cytokine included in the recombinant oncolytic virus provided in each of Preparation Examples 48-65 was IL-15 including an amino acid sequence as shown in SEQ ID NO 35. See Table 3 for details.

The cytokine included in the recombinant oncolytic virus provided in each of Preparation Examples 66-83 was IL-18 including an amino acid sequence as shown in SEQ ID NO 36. See Table 3 for details.

I. The construction method of the recombinant oncolytic viruses provided in said preparation examples is the same as the construction method in Preparation Example 9, except that:

the cytokines as shown in Table 3 were further introduced by PCR during insertion of exogenous gene in step (2). The tumor antigen and the cytokine were inserted between the G and L proteins. With regard to the order of insertion of the tumor antigen and the cytokine, the cytokine may be inserted first, then the tumor antigen; or the tumor antigen may be inserted first, then the cytokine. In the present application, the cytokine was inserted first, then the tumor antigen.

Step (4) further includes sequencing of the cytokine. The viral genomic RNA was extracted using a Trizol kit, and reverse transcription was performed using random primers. The reverse transcribed cDNA was subjected to PCR using primers designed for the gene sequence of the cytokine.

The primer sequences designed for the gene sequence encoding the cytokines were:

IL12 F: CCCTCGAGATGTGGCCCCCTGGGT,
IL12 R: CGGCTAGCTTAACTGCAGGGCACAGATG.
IL-15 F: CCGCTCGAGATGAGAATTTCGAAACC,
IL-15 R: CGGCTAGCTCAAGAAGTGTTGATGAAC.
IL-18 F: CCCTCGAGATGGCTGCTGAACCAGTAG,
IL-18 R: CGGCTAGCCTAGTCTTCGTTTTGAAC.

The product was extracted after 1% agarose gel electrophoresis and sequenced by a sequencing company. The sequencing results are shown in Table 3.

II. The construction method of immune cells provided in said preparation examples is the same as above.

Table 3 Mutations of the recombinant oncolytic viruses in Preparation Examples 30-83

| Prep arati on | Mutated sites of proteins | Tumor antigen/cytokine | | | Immune cell |
|---|---|---|---|---|---|
| | | Type | Typ | Sequ | Type |

| Example | | | e | ence | |
|---|---|---|---|---|---|
| 30 | M protein: N32S, N49D, M51R, H54Y, V221F, V225I, S226R.<br>G protein: V53I, A141V, D172Y, K217E, D232G, V331A, V371E, G436D, T438S, F453L, T471I, Y487H.<br>N protein: I14V, R155K, S353N.<br>P protein: R50K, V76A, D99E, L126S, L140S, H151Y, I168M, K170E, Y189S, N237D.<br>L protein: S87P, I487T. | CD19 | IL-12 | SEQ ID NO 33, SEQ ID NO 34 | CD19-CAR-T cell |
| 31 | | CD22 | | | CD22-CAR-T cell |
| 32 | | BCMA | | | BCMA-CAR-T cell |
| 33 | | NY-ESO-1 | | | NY-ESO-1 TCR-T cell |
| 34 | | MAGE A4 | | | MAGE A4 TCR-T cell |
| 35 | | cMet | | | cMet -CAR-T cell |
| 36 | | Claude 18.2 | | | Claude 18.2-CAR-T cell |
| 37 | | MSLN | | | MSLN-CAR-T cell |
| 38 | | EGFR | | | EGFR-CAR-T cell |
| 39 | | VEGFR2 | | | VEGFR2-CAR-T cell |
| 40 | | HER-2 | | | HER-2-CAR-T cell |
| 41 | | TPBG | | | TPBG-CAR-T cell |
| 42 | | CD19 | | | CD19-CAR-γδ-T cell |
| 43 | | CD19 | | | CD19-CAR-Crispered -T cell |
| 44 | | EGFR | | | STAR-T- EGFR cell |
| 45 | | CD19 | | | CD19-CAR-NK cell |
| 46 | | CD19 | | | CD19-IPSC CAR-NK cell |
| 47 | | CD19 | | | CD19-CAR-M cell |
| 48 | M protein: N32S, N49D, M51R, H54Y, V221F, V225I, S226R.<br>G protein: V53I, A141V, D172Y, K217E, D232G, V331A, V371E, G436D, T438S, F453L, T471I, Y487H.<br>N protein: I14V, R155K, S353N.<br>P protein: R50K, V76A, D99E, L126S, L140S, H151Y, I168M, K170E, Y189S, N237D.<br>L protein: S87P, I487T. | CD19 | IL-15 | SEQ ID NO 35 | CD19-CAR-T cell |
| 49 | | CD22 | | | CD22-CAR-T cell |
| 50 | | BCMA | | | BCMA-CAR-T cell |
| 51 | | NY-ESO-1 | | | NY-ESO-1 TCR-T cell |
| 52 | | MAGE A4 | | | MAGE A4 TCR-T cell |
| 53 | | cMet | | | cMet -CAR-T cell |
| 54 | | Claude 18.2 | | | Claude 18.2-CAR-T cell |
| 55 | | MSLN | | | MSLN-CAR-T cell |
| 56 | | EGFR | | | EGFR-CAR-T cell |
| 57 | | VEGFR2 | | | VEGFR2-CAR-T cell |
| 58 | | HER-2 | | | HER-2-CAR-T cell |

| | | | | |
|---|---|---|---|---|
| 59 | | TPBG | | TPBG-CAR-T cell |
| 60 | | CD19 | | CD19-CAR-γδ-T cell |
| 61 | | CD19 | | CD19-CAR-Crispere d -T cell |
| 62 | | EGFR | | STAR-T- EGFR cell |
| 63 | | CD19 | | CD19-CAR-NK cell |
| 64 | | CD19 | | CD19-IPSC CAR-NK cell |
| 65 | | CD19 | | CD19-CAR-M cell |
| 66 | M protein: N32S, N49D, M51R, H54Y, V221F, V225I, S226R.<br>G protein: V53I, A141V, D172Y, K217E, D232G, V331A, V371E, G436D, T438S, F453L, T471I, Y487H.<br>N protein: I14V, R155K, S353N.<br>P protein: R50K, V76A, D99E, L126S, L140S, H151Y, I168M, K170E, Y189S, N237D.<br>L protein: S87P, I487T. | CD19 | IL-18 | CD19-CAR-T cell |
| 67 | | CD22 | | CD22-CAR-T cell |
| 68 | | BCMA | | BCMA-CAR-T cell |
| 69 | | NY-ESO-1 | | NY-ESO-1 TCR-T cell |
| 70 | | MAGE A4 | | MAGE A4 TCR-T cell |
| 71 | | cMet | | cMet -CAR-T cell |
| 72 | | Claude 18.2 | | Claude 18.2-CAR-T cell |
| 73 | | MSLN | SEQ ID NO 36 | MSLN-CAR-T cell |
| 74 | | EGFR | | EGFR-CAR-T cell |
| 75 | | VEGFR 2 | | VEGFR2-CAR-T cell |
| 76 | | HER-2 | | HER-2-CAR-T cell |
| 77 | | TPBG | | TPBG-CAR-T cell |
| 78 | | CD19 | | CD19-CAR-γδ-T cell |
| 79 | | CD19 | | CD19-CAR-Crispere d -T cell |
| 80 | | EGFR | | STAR-T- EGFR cell |
| 81 | | CD19 | | CD19-CAR-NK cell |
| 82 | | CD19 | | CD19-IPSC CAR-NK cell |
| 83 | | CD19 | | CD19-CAR-M cell |

Preparation Examples 84-95

Preparation Examples 84-95 respectively provide a method for treating tumors using an oncolytic virus vaccine.

The recombinant oncolytic viruses in said preparation examples 84-95 were the oncolytic virus vaccines in Preparation Examples 9 and 13-29, respectively. See Table 4 for details. The difference lies in the types of the tumor antigen.

Table 4 Mutations of the recombinant oncolytic viruses in Preparation Examples 84-95

| Preparation Example | M protein | | Tumor antigen | |
|---|---|---|---|---|
| | Mutated sites and amino acids after mutation | | Type | Sequence |
| 84 | | | CD19 | SEQ ID NO 20 |
| 85 | | | CD22 | SEQ ID NO 21 |
| 86 | | | BCMA | SEQ ID NO 22 |
| 87 | M protein: N32S, N49D, M51R, H54Y, V221F, V225I, S226R. Y487H. | | NY-ESO -1 | SEQ ID NO 24 |
| 88 | G protein: V53I, A141V, D172Y, K217E, D232G, V331A, V371E, G436D, T438S, F453L, T471I, | | MAGE A4 | SEQ ID NO 25 |
| 89 | | | cMet | SEQ ID NO 26 |
| 90 | N protein: I14V, R155K, S353N. | | Claude 18.2 | SEQ ID NO 27 |
| 91 | P protein: R50K, V76A, D99E, L126S, L140S, H151Y, I168M, K170E, Y189S, N237D. | | MSLN | SEQ ID NO 28 |
| 92 | | | EGFR | SEQ ID NO 29 |
| 93 | L protein: S87P, I487T. | | VEGFR 2 | SEQ ID NO 30 |
| 94 | | | HER-2 | SEQ ID NO 31 |
| 95 | | | TPBG | SEQ ID NO 32 |

Preparation Examples 96-113

Preparation Examples 96-113 respectively provide a method for treating tumors using immune cells.

The immune cells in said preparation examples 96-113 were the immune cells in Preparation Examples 9 and 13-29, respectively. See Table 5 for details. The difference lies in the types of immune cells and the chimeric antigen receptors.

Table 5 Immune cells in Preparation Examples 96-113

| Preparation Example | Type | Preparation Example | Type |
|---|---|---|---|
| 96 | CD19-CAR-T cell | 105 | VEGFR2-CAR-T cell |
| 97 | CD22-CAR-T cell | 106 | HER-2-CAR-T cell |
| 98 | BCMA-CAR-T cell | 107 | TPBG-CAR-T cell |
| 99 | NY-ESO-1 TCR-T cell | 108 | CD19-CAR-$\gamma\delta$-T cell |
| 100 | MAGE A4 TCR-T cell | 109 | CD19-CAR-Crispered -T cell |
| 101 | cMet -CAR-T cell | 110 | STAR-T- EGFR cell |
| 102 | Claude 18.2-CAR-T cell | 111 | CD19-CAR-NK cell |
| 103 | MSLN-CAR-T cell | 112 | CD19-IPSC CAR-NK cell |
| 104 | EGFR-CAR-T cell | 113 | CD19-CAR-M cell |

**EXAMPLE**

In this example, the killing ability of the methods for treating tumors using a combination of an oncolytic virus vaccine and/or immune cells provided in Preparation Examples 9-113 to different cells in vitro were tested.

The test method used was CCK8 assay. That is, the oncolytic virus vaccines and/or immune cells provided in Preparation Examples 9-113 and wild-type oncolytic virus were added to the culture medium of different cells respectively, and cell activity was detected by MTT assay after 24 h.

The cells tested included: LLC cell and MEF cell.

I. Oncolytic virus vaccines and immune cells provided in Preparation Examples 9-83-Specific test method was as follows:

(1) 100 $\mu$L of Vero (LLC/MEF) cell suspension was added to each well of 96-well plates, with $4 \times 10^3$ cells/well; the 96-well culture plates were placed in an environment of 37 °C and 5% $CO_2$ for incubation for 16 h.

(2) The oncolytic virus vaccines prepared in said preparation examples were diluted to four gradients with MOI (multiplicity of infection) of 0.001, 0.01, 0.1, and 1.0, respectively; and the diluted recombinant oncolytic viruses of all the dilution gradients were seeded into the 96-well plates from step (1), 100 $\mu$l/well, 3 wells for each dilution gradient.

Moreover, the immune cells were diluted to four gradients of 1:1, 5:1, 10:1 and 20:1 according to the effect-target ratio (E:T); the immune cells of all the dilution gradients were seeded into the 96-well culture plates from step (1) respectively, 100 $\mu$L/well, 3 wells for each dilution gradient; and the 96-well cultures was placed in an environment of 37 °C and 5% $CO_2$ for incubation for 48 h.

(3) Cell supernatants were removed from the 96-well culture plates from step (2), fresh culture medium of DMED was then added to the 96-well plates, 100 $\mu$L/well; then, CCK8 solution was added, 10 $\mu$L/well; and the 96-well plates were then placed in an environment of 37 °C and 5% $CO_2$ for incubation for 4 h.

(4) The 96-well plates were centrifuged at room temperature, 2500 rpm/min for 5 min and the supernatants were gently removed with a 1 mL disposable sterile syringe; then, DMSO was added to each well of the 96-well plates, 100 $\mu$l/well; and the 96-well plates were then placed at 37 °C for 10 min. Using a multifunctional microplate reader, the 96-well plates were shaken for 2 min and the OD value of each well in the 96-well plates was measured at a wavelength of 570 nm or 490 nm, and the tumor cell killing rate was calculated.

Tumor cell killing rate (%)=(OD value of tumor cell control group-OD value of experimental group)/OD value of tumor cell control group $\times$100%.

II. Oncolytic virus vaccines provided in Preparation Examples 84-95 and wild-type oncolytic virus -Specific test method was as follows:

(1) 100 $\mu$L of Vero (LLC/MEF) tumor cell suspension was added to each well of 96-well plates, with $4 \times 10^3$ cells/well; the 96-well culture plates were placed in an environment of 37 °C and 5% $CO_2$ for incubation for 16 h.

(2) The oncolytic virus vaccines prepared in said preparation examples and wild-type oncolytic virus were diluted to four gradients with MOI of 0.001, 0.01, 0.1, and 1.0, respectively; and the diluted oncolytic virus vaccines and wild-type oncolytic virus of all the dilution gradients were seeded into the 96-well plates from step (1), 100 $\mu$l/well, 3 wells for each dilution gradient; the 96-well culture plates were then placed in an environment of 37 °C and 5% $CO_2$ for incubation for 48 h.

(3) Supernatants were removed from the 96-well culture plates from step (2), fresh culture medium of DMED was then added to the 96-well plates, 100 $\mu$L/well; then, CCK8 solution was added, 10 $\mu$L/well; and the 96-well plates were then placed in an environment of 37 °C and 5% $CO_2$ for incubation for 4 h.

(4) The 96-well plates were centrifuged at room temperature, 2500 rpm/min for 5 min and the supernatants were gently removed with a 1 mL disposable sterile syringe; then, DMSO was added to each well of the 96-well plates, 100 $\mu$l/well; and the 96-well plates were then placed at 37 °C for 10 min. Using a multifunctional microplate reader, the 96-well plates were shaken for 2 min and the OD value of each well in the 96-well plates was measured at a wavelength of 570 nm or 490 nm, and the tumor cell killing rate was calculated.

III. Immune cells provided in Preparation Examples 96-113-Specific test method was as follows:

(1) 100 $\mu$L of Vero (LLC/MEF) tumor cell suspension was added to each well of 96-well plates, with $4 \times 10^3$

cells/well; the 96-well culture plates were placed in an environment of 37 °C and 5% $CO_2$ for incubation for 16 h.

(2) The immune cells were diluted to four gradients of 1:1, 5:1, 10:1 and 20:1 according to the effect-target ratio (E:T); the immune cells of all the dilution gradients were seeded into the 96-well culture plates from step (1) respectively, 100 μL/well, 3 wells for each dilution gradient; and the 96-well cultures was placed in an environment of 37 °C and 5% $CO_2$ for incubation for 48 h.

(3) Supernatants were removed from the 96-well culture plates from step (2), fresh culture medium of DMED was then added to the 96-well plates, 100 μL/well; then, CCK8 solution was added, 10 μL/well; and the 96-well plates were then placed in an environment of 37 °C and 5% $CO_2$ for incubation for 4 h.

(4) The 96-well plates were centrifuged at room temperature, 2500 rpm/min for 5 min and the supernatants were gently removed with a 1 mL disposable sterile syringe; then, DMSO was added to each well of the 96-well plates, 100 μl/well; and the 96-well plates were then placed at 37 °C for 10 min. Using a multifunctional microplate reader, the 96-well plates were shaken for 2 min and the OD value of each well in the 96-well plates was measured at a wavelength of 570 nm or 490 nm, and the tumor cell killing rate was calculated.

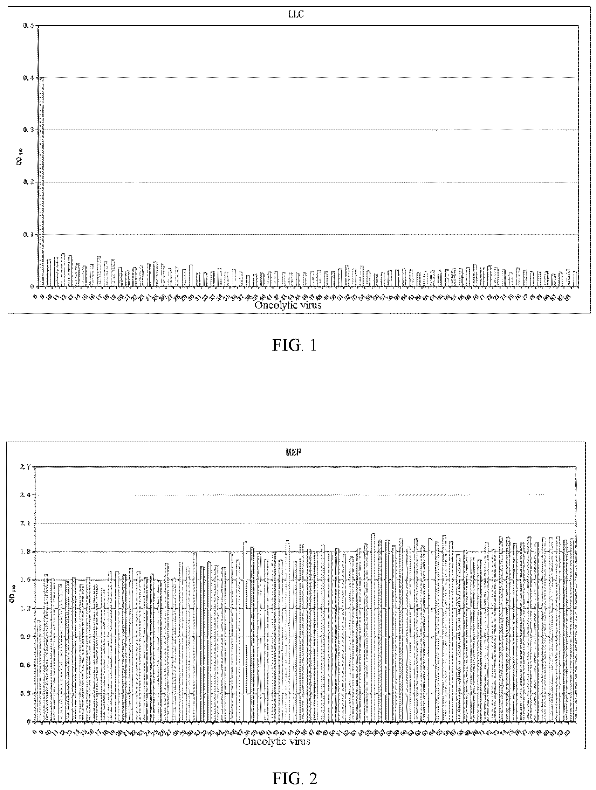

The test results are shown in FIGS. 1-6. As shown in the figures, the number 0 on the horizontal axis represents the wild-type oncolytic virus; the numbers 9-113 on the horizontal axis represent the recombinant oncolytic viruses prepared in Preparation Examples 9-113, respectively; $OD_{570}$ on the vertical axis represents the OD values of the cells. The larger the value of $OD_{570}$, the poorer the killing ability of a recombinant oncolytic virus to the cell; the smaller the value of $OD_{570}$, the better the killing ability of a recombinant oncolytic virus to the cell.

FIG. 1 shows the test result of the killing ability of the method for treating tumors using a combination of the oncolytic virus vaccines prepared in Preparation Examples 9-83 of the present application and immune cells to LLC cells in vitro and the killing ability of a wild-type oncolytic virus to LLC cells in vitro.

FIG. 2 shows the test result of the killing ability of the method for treating tumors using a combination of the oncolytic virus vaccines prepared in Preparation Examples 9-83 of the present application and immune cells to MEF cells in vitro and the killing ability of a wild-type oncolytic virus to MEF cells in vitro.

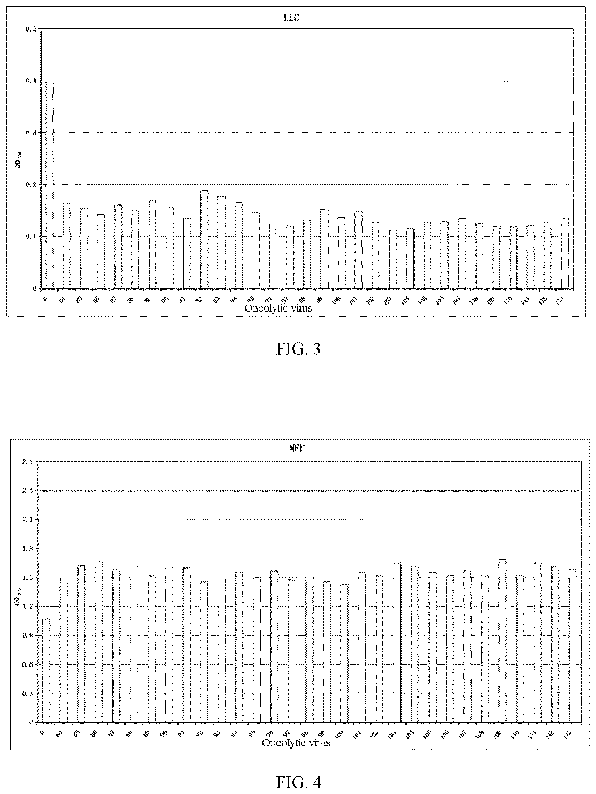

FIG. 3 shows the test result of the killing ability of the oncolytic virus vaccines prepared in Preparation Examples 84-95 of the present application, the immune cells prepared in Preparation Examples 96-113 of the present application, and a wild-type oncolytic virus to LLC cells in vitro.

FIG. 4 shows the test result of the killing ability of the oncolytic virus vaccines prepared in Preparation Examples 84-95 of the present application, the immune cells prepared in Preparation Examples 96-113 of the present application, and a wild-type oncolytic virus to MEF cells in vitro.

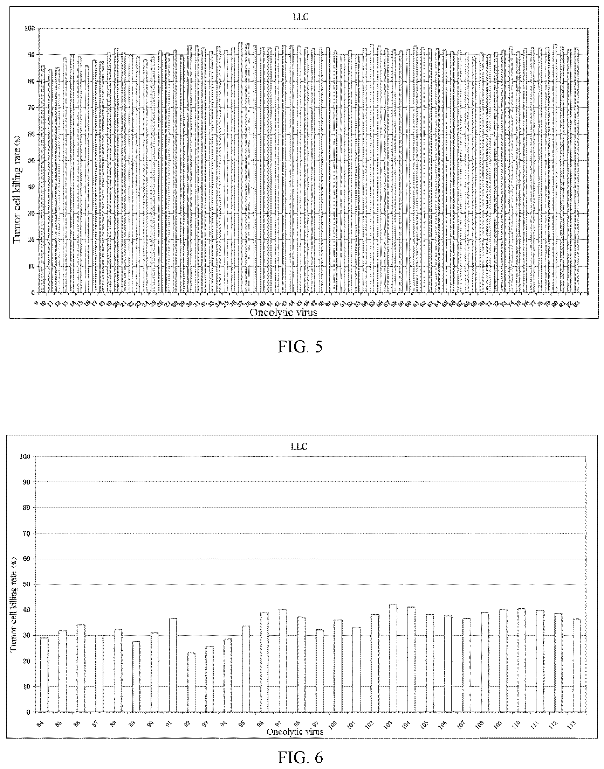

FIG. 5 shows tumor cell killing rates of the method for treating tumors using a combination of the oncolytic virus vaccines prepared in Preparation Examples 9-83 of the present application and immune cells.

FIG. 6 shows tumor cell killing rates of the oncolytic virus vaccines prepared in Preparation Examples 84-95 of the present application and the immune cells prepared in Preparation Examples 96-113 of the present application.

It can be seen from the above figures that the method for treating tumors using the oncolytic virus vaccines provided in Preparation Examples 1-83 of the present application in combination with immune cells has better killing ability to LLC cells in vitro, which is superior to the killing ability of the oncolytic virus vaccines provided in Preparation Examples 84-95 and the immune cells provided in Preparation Examples 96-113 to LLC cell in vitro.

From the above test results, it can be seen that the method for treating tumors using a combination of an oncolytic virus vaccine and immune cells provided by the present application has better killing ability to LLC cells in vitro. It can be concluded that the method for treating tumors using a combination of an oncolytic virus vaccine and immune cells provided by the present application has better killing ability to cancer cells (such as 4T1 cell, MC38 cell and Hela cell) in vitro. Moreover, the method for treating tumors using a combination of an oncolytic virus vaccine and immune cells provided by the present application almost has no effect to MEF cells, indicating that the method for treating tumors using a combination of an oncolytic virus vaccine and immune cells provided by the present application can be better used for damaging and killing abnormal cells such as tumor and cancer cells, without causing damage to normal cells. Therefore, the method for treating tumors using a combination of an oncolytic virus vaccine and immune cells provided by the present application ensures safety to normal cells and killing ability to tumor and cancer cells, thereby achieving broad clinical application prospects.

The specific examples are merely an explanation of the present application and not for limiting the present application. Those skilled in the art may make modifications, without creative contribution, to the examples as needed after reading this specification. Any of the modifications made within the scope of the claims of the present application shall be protected by the Patent Law.

Claims

1. A method for treating tumors using a combination of an oncolytic virus vaccine and immune cells, **characterized in that** the immune cells and the oncolytic virus vaccine are used in combination for treating the tumors;

   the oncolytic virus vaccine comprises a recombinant oncolytic virus expressing a tumor antigen and is used for targeting tumor cells;
   the immune cells express a chimeric antigen receptor paired with the tumor antigen, and are used for killing or destroying the tumor cells targeted;
   the recombinant oncolytic virus comprises an M protein, a G protein, an N protein, a P protein, and an L protein; compared to an amino acid sequence shown in SEQ ID NO 1,
   the M protein comprises site mutations of M51R, V221F and S226R; or
   the M protein comprises site mutations of N32S, N49D, M51R, H54Y, V221F, V225I and S226R; or
   the M protein comprises site mutations of N32S, N49D, M51R, H54Y, knockouting of bases encoding leucine at position 111, V221F, V225I and S226R; or
   the M protein comprises site mutations of N32S, N49D, M51R, H54Y, L111A, V221F, V225I and S226R; or
   the M protein comprises site mutations of G21E, N32S, N49D, M51R, H54Y, V221F, V225I and S226R; or
   the M protein comprises site mutations of G21E, N32S, M33A, N49D, M51R, H54Y, V221F, V225I and S226R; or
   the M protein comprises site mutations of G21E, N32S, M33A, N49D, M51R, H54Y, A133T, V221F, V225I and S226R; or
   the M protein comprises site mutations of N32S, M33A, N49D, M51R, H54Y, V221F, V225I and S226R; or
   the M protein comprises site mutations of N32S, M33A, N49D, M51R, H54Y, A133T, V221F, V225I and S226R; or
   the M protein comprises site mutations of N32S, N49D, M51R, H54Y, A133T, V221F, V225I, and S226R;
   compared to an amino acid sequence shown in SEQ ID NO 12, the G protein comprises site mutations of V53I, A141V, D172Y, K217E, D232G, V331A, V371E, G436D, T438S, F453L, T471I, and Y487H;
   compared to an amino acid sequence shown in SEQ ID NO 14, the N protein comprises site mutations of I14V, R155K, and S353N;
   compared to an amino acid sequence shown in SEQ ID NO 16, the P protein comprises site mutations of R50K, V76A, D99E, L126S, L140S, H151Y, I168M, K170E, Y189S, and N237D; and
   compared to an amino acid sequence shown in SEQ ID NO 18, the L protein comprises site mutations of S87P and I487T.

2. The method for treating tumors using a combination of an oncolytic virus vaccine and immune cells according to claim 1, **characterized in that** the recombinant oncolytic virus comprises a rhabdovirus.

3. The method for treating tumors using a combination of an oncolytic virus vaccine and immune cells according to claim 2, **characterized in that** the rhabdovirus comprises a vesicular stomatitis virus (VSV).

4. The method for treating tumors using a combination of an oncolytic virus vaccine and immune cells according to claim 1, **characterized in that** the tumor antigen is selected from a hematological tumor antigen and a solid tumor antigen.

5. The method for treating tumors using a combination of an oncolytic virus vaccine and immune cells according to claim 4, **characterized in that**

   the solid tumor antigen comprises but is not limited to 5T4, RORI, EGFR, FcγRI, FcγRIIa, FcγRIIb, CD28, CD137, CTLA-4, HER-2, FAS, FAP, LGR5, C5aR1, A2AR, FGFR1, FGFR2, FGFR3, FGFR4, glucocorticoid-induced TNFR-related (GITR) protein, LTβR, TRAIL receptor 1, TRAIL receptor 2, prostate-specific membrane antigen (PSMA) protein, prostate stem cell antigen (PSCA) protein, tumor-related protein carbonic anhydrase IX (CAIX), EGFR1, EGFRvIII, ErbB3, folate receptor, ephrin receptor, PDGFRa, ErbB-2, CD2, CD40, CD74, CD80, CD86, CCAM5, CCAM6, p53, cMet, HGFR, MAGE-A1, MAGE-A2, MAGE-A3, MAGE-A4, MAGE-A6, MAGE-A10, MAGE-A12, BACE, DAM-6, DAM-10, GAGE-1, GAGE-2, GAGE-8, GAGE-3, GAGE-4, GAGE-5, GAGE-6, GAGE-7B, NA88-A, NY-ESO-1, BRCA1, BRCA2, MART-1, MCIR, Gp100, PSA, PSM, tyrosinase, TRP-1, TRP-2, ART-4, CAMEL, Cyp-B, hTERT, hTRT, iCE, MUC2, P-cadherin, myostatin, Cripto, MUC5AC, PRAME, P15, RU1, RU2, SART-1, SART-3, AFP, β-catenin/m, caspase-8/m, CDK-4/m, ELF2M, GnT-V, G250, HSP70-2M, HST-2, KIAA0205, MUM-1, MUM-2, MUM-3, myosin/m, RAGE, SART-2, TRP-2/INT2, 707-AP, annexin II, CDC27/m, TPI/mbcr-abl, ETV6/AML, LDLR/FUT, Pml/RARa, TEL/AML1, CD28, CD137, CanAg, mesothelin (MSLN), DR5, PD-1, PD-L1, IGF-1R, CXCR4, neuropilin 1 (NRP-1), glypican, EphA2, B7-H3, B7-H4, gpA33, GPC3, SSTR2, GD2, VEGF-A, VEGFR-2, PDGFR-a, ANKL, RANKL, MSLN, EBV, TROP2, FOLR1, AXL, Claude 18.2, MUC1, and TPBG;

the hematological tumor antigen comprises butis not limited to BCMA, CD4, CD5, CD7, CD10, FcγRIIIa, FcγRIIIb, CD19, CD20, CD22, CD23, CD30, CD33, CD34, CD37, CD38, CD44, CD47, CD56, CD70, CD117, CD123, CD138, CD174 , CLL-1, ROR1, NKG2DL1/2, IL1R3, FCRL5, GPRC5D, CLEC12A, WT1, FLT3, TLR8, SHP2, KAT6A/B, CSNK1A1, FLI1, IKZF1/3, PI3K, c-Kit , SLAMF3, SLAMF7, TCR B-chain, ITGB7, k-1gG, TACI, TRBCI, and LeY.

6. The method for treating tumors using a combination of an oncolytic virus vaccine and immune cells according to claim 5, **characterized in that** the tumor antigen is one or more selected from a group consisting of: CD19, CD22, BCMA, MUC1, NY-ESO-1, MAGE A4, cMet, Claude 18.2, MSLN, EGFR, VEGFR2, HER-2, TPBG, AFP, and MAGE-A10.

7. The method for treating tumors using a combination of an oncolytic virus vaccine and immune cells according to claim 1, **characterized in that** the recombinant oncolytic virus expresses at least one or more of the tumor antigen.

8. The method for treating tumors using a combination of an oncolytic virus vaccine and immune cells according to claim 1, **characterized in that** the immune cells are selected from T cells, NK cells and M cells.

9. The method for treating tumors using a combination of an oncolytic virus vaccine and immune cells according to claim 1, **characterized in that** the immune cells are selected from CAR-T cells, TCR-T cells, CAR-γδ-T cells, CAR-Crispered-T cells, STAR-T cells, CAR-NK cells, and CAR-M cells.

10. The method for treating tumors using a combination of an oncolytic virus vaccine and immune cells according to claim 1, **characterized in that** the immune cells are selected from autologous cells, allogeneic cells, or immune cells derived from IPSC.

11. The method for treating tumors using a combination of an oncolytic virus vaccine and immune cells according to claim 1, **characterized in that** the immune cells comprise immune cells obtained by means of in vitro expansion.

12. The method for treating tumors using a combination of an oncolytic virus vaccine and immune cells according to claim 1, **characterized in that** the immune cells express at least one or more of the antigen receptor.

13. The method for treating tumors using a combination of an oncolytic virus vaccine and immune cells according to claim 1, **characterized in that** the recombinant oncolytic virus further comprises a cytokine encoded by an exogenous gene.

14. The method for treating tumors using a combination of an oncolytic virus vaccine and immune cells according to claim 13, **characterized in that** the cytokine is selected from interleukins (IL), interferons (IFN), tumor necrosis factors (TNF), colony stimulating factors (CSF), transforming growth factor-β family (TGF-β family) and chemokine family.

15. The method for treating tumors using a combination of an oncolytic virus vaccine and immune cells according to claim 14, **characterized in that** the cytokine is one or more selected from a group consisting of: GM-CSF, G-CSF, M-CSF, IL-1, IL-2, IL-4, IL-5, IL-6, IL-9, IL-10, IL-12, IL-13, IL-15, IL-17, IL-18, IL-23, IL-27, IFN-a, IFN-β, IFN-γ, IFN-β, TGF-β, and TNF-α.

16. The method for treating tumors using a combination of an oncolytic virus vaccine and immune cells according to claim 15, **characterized in that** the cytokine is one or more selected from a group consisting of: GM-CSF, IL-2, IL-12, IL-15, IL-18, IFN-β, and TNF-α.

17. The method for treating tumors using a combination of an oncolytic virus vaccine and immune cells according to claim 1, **characterized in that** the recombinant oncolytic virus comprises a nucleic acid molecule; the nucleic acid molecule comprises a nucleic acid sequence encoding the M protein with the site mutations, a nucleic acid sequence encoding the G protein with the site mutations, a nucleic acid sequence encoding the N protein with the site mutations, a nucleic acid sequence encoding the P protein with the site mutations, a nucleic acid sequence encoding the L protein with the site mutations, a nucleic acid sequence encoding the tumor antigen, and a nucleic acid sequence encoding the cytokine.

18. The method for treating tumors using a combination of an oncolytic virus vaccine and immune cells according to claim 17, **characterized in that** in the nucleic acid molecule, the nucleic acid sequence encoding the tumor antigen is located between the nucleic acid sequence encoding the G protein with the site mutations and the nucleic acid sequence encoding the L protein with the site mutations.

19. The method for treating tumors using a combination of an oncolytic virus vaccine and immune cells according to claim 17, **characterized in that** in the nucleic acid molecule, the nucleic acid sequence encoding the cytokine is located between the nucleic acid sequence encoding the G protein with the site mutations and the nucleic acid sequence encoding the L protein with the site mutations.

20. The method for treating tumors using a combination of an oncolytic virus vaccine and immune cells according to claim 17, **characterized in that** in the nucleic acid molecule, the nucleic acid sequence encoding the cytokine is located between the nucleic acid sequence encoding the tumor antigen and the nucleic acid sequence encoding the L protein with the site mutations, or between the nucleic acid sequence encoding the G protein with the site mutations and the nucleic acid sequence encoding the tumor antigen.

21. The method for treating tumors using a combination of an oncolytic virus vaccine and immune cells according to claim 1, **characterized in that** the method for treating tumors using a combination of an oncolytic virus vaccine and immune cells is used for killing an abnormally proliferative cell in a sustained manner.

22. The method for treating tumors using a combination of an oncolytic virus vaccine and immune cells according to claim 21, **characterized in that** the abnormally proliferative cell is selected from tumor cells or tumor tissue-related cells.

23. The method for treating tumors using a combination of an oncolytic virus vaccine and immune cells according to claim 21, **characterized in that** the tumors comprise solid tumors or hematological tumors.

24. The method for treating tumors using a combination of an oncolytic virus vaccine and immune cells according to claim 21, **characterized in that** the tumors comprise but are not limited to acute lymphoblastic leukemia, acute B-cell leukemia, chronic non-lymphocytic leukemia, non-Hodgkin's lymphoma, anal cancer, astrocytoma, basal cell carcinoma, bile duct cancer, bladder cancer, mastocarcinoma, breast cancer, cervical cancer, chronic myeloproliferative tumors, colorectal cancer, endometrial cancer, ependymoma, esophageal cancer, diffuse large B-cell lymphoma, sensorineuroblastoma, Ewing's sarcoma, fallopian tube cancer, gallbladder cancer, gastric cancer, gastrointestinal carcinoid tumors, hepatocellular carcinoma, hypopharyngeal cancer, Kaposi sarcoma, kidney cancer, Langerhans cell hyperplasia, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumors, pharyngeal cancer, pituitary tumors, prostate cancer, rectal cancer, renal cell carcinoma, retinoblastoma, skin cancer, small cell lung cancer, small intestine cancer, squamous neck cancer, testicular cancer, thymoma, thyroid cancer, uterine cancer, vaginal cancer and vascular tumors.

25. A composition, **characterized in that** the composition comprises the oncolytic virus vaccine and immune cells according to claim 1.

FIG. 1

FIG. 2

FIG. 3

FIG. 4

FIG. 5

FIG. 6

## INTERNATIONAL SEARCH REPORT

| International application No. |
| --- |
| **PCT/CN2024/082160** |

| A. | CLASSIFICATION OF SUBJECT MATTER |
| --- | --- |

A61K39/00(2006.01)i; A61K35/766(2015.01)i; A61K38/19(2006.01)i; A61K38/20(2006.01)i; A61P35/00(2006.01)i; A61P35/02(2006.01)i

According to International Patent Classification (IPC) or to both national classification and IPC

| B. | FIELDS SEARCHED |
| --- | --- |

Minimum documentation searched (classification system followed by classification symbols)

IPC: A61K; A61P

Documentation searched other than minimum documentation to the extent that such documents are included in the fields searched

Electronic data base consulted during the international search (name of data base and, where practicable, search terms used)

CNTXT, WPABSC, ENTXTC, ENTXT, USTXT, WOTXT, CJFD, VEN, CNKI, DWPI, 万方, WANFANG, 百度, Baidu, bing, PUBMED, ISI Web of Knowledge, NCBI GenBank, EBI-EMBL, STN, 读秀, DUXIU: SEQ ID Nos: 1-73, 溶瘤病毒, 疫苗, 免疫细胞, 联合, 治疗, 肿瘤, 重组, 突变, 取代, 氨基酸, G21E, N32S, M33A, N49D, M51R, M51A, H54Y, L111A, A133T, V221F, V225I, S226R, V53I, A141V, D172Y, K217E, D232G, V331A, V371E, G436D, T438S, F453L, T471I, Y487H, I14V, R155K, S353N, R50K, V76A, D99E, L126S, L140S, H151Y, I168M, K170E, Y189S, N237D, S87P, I487T, tumor, cancer, CAR, CAR-T, TCR, carcinoma, Oncolytic, viruses, vaccines, immune cells, combinations, therap+, recombinant, mutations, substitutions, amino acids.

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 116350759 A (SHANGHAI RONGRUI PHARMACEUTICAL TECHNOLOGY CO., LTD.) 30 June 2023 (2023-06-30) claims 1-25 | 1-25 |
| Y | CN 111286493 A (SHANGHAI RONGRUI PHARMACEUTICAL TECHNOLOGY CO., LTD.) 16 June 2020 (2020-06-16) claims 1-11, and description, paragraphs 10-22 | 1-25 |
| Y | CN 114540316 A (SHANGHAI RONGRUI PHARMACEUTICAL TECHNOLOGY CO., LTD.) 27 May 2022 (2022-05-27) claims 1-43, and description, paragraphs 2, 5, 236 and 267 | 1-25 |
| Y | CN 111320677 A (SUZHOU AOTEMING MEDICINE TECHNOLOGY CO., LTD. et al.) 23 June 2020 (2020-06-23) claims 26-60 | 1-25 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **14 June 2024** | **25 June 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/082160** |

### C. DOCUMENTS CONSIDERED TO BE RELEVANT

| Category* | Citation of document, with indication, where appropriate, of the relevant passages | Relevant to claim No. |
|---|---|---|
| A | US 2022016191 A1 (MASSACHUSETTS INSTITUTE OF TECHNOLOGY) 20 January 2022 (2022-01-20)<br>    entire document | 1-25 |
| A | TIAN, Y. et al. "Engineering Strategies to Enhance Oncolytic Viruses in Cancer Immunotherapy"<br>*Signal Transduction and Targeted Therapy*, 06 April 2022 (2022-04-06),<br>    pp. 1-24 | 1-25 |

Form PCT/ISA/210 (second sheet) (July 2022)

## INTERNATIONAL SEARCH REPORT

International application No.

**PCT/CN2024/082160**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
|---|
| **PCT/CN2024/082160** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **1-24**
   because they relate to subject matter not required to be searched by this Authority, namely:

   The subject matter of claims 1-24 relates to a method for treatment of a human or animal body, and therefore an international search is not performed according to PCT Rule 39.1(iv). The present search is provided on the basis of the use of an oncolytic virus vaccine and an immune cell in the preparation of drugs used in combination for treating tumors.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

INTERNATIONAL SEARCH REPORT
Information on patent family members

International application No.

**PCT/CN2024/082160**

| Patent document cited in search report | | | Publication date (day/month/year) | Patent family member(s) | | | Publication date (day/month/year) |
|---|---|---|---|---|---|---|---|
| CN | 116350759 | A | 30 June 2023 | None | | | |
| CN | 111286493 | A | 16 June 2020 | WO | 2021228105 | A1 | 18 November 2021 |
| | | | | US | 2023256079 | A1 | 17 August 2023 |
| | | | | JP | 2023524915 | A | 13 June 2023 |
| | | | | JP | 7468958 | B2 | 16 April 2024 |
| | | | | TW | 202142687 | A | 16 November 2021 |
| | | | | AU | 2021271961 | A1 | 15 December 2022 |
| | | | | EP | 4141111 | A1 | 01 March 2023 |
| | | | | EP | 4141111 | A4 | 20 December 2023 |
| | | | | CA | 3178631 | A1 | 18 November 2021 |
| | | | | KR | 20230002564 | A | 05 January 2023 |
| CN | 114540316 | A | 27 May 2022 | None | | | |
| CN | 111320677 | A | 23 June 2020 | None | | | |
| US | 2022016191 | A1 | 20 January 2022 | WO | 2022015407 | A1 | 20 January 2022 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015036583 A

- CN 114958771 A